# EUROPEAN PATENT APPLICATION

(11) **EP 2 269 567 A1**
(43) Date of publication of application: **05.01.2011**
(21) Application number: 09008543.2
(22) Date of filing: 30.06.2009
(51) Int. Cl.: A61K 8/37, C07C 67/03, C07C 67/08, A61Q 1/02, A61Q 1/06, A61Q 5/02, A61Q 5/12, A61Q 15/00, A61Q 17/04, A61Q 19/00

(54) **Ester mixtures and compositions comprising such ester mixtures**

(71) Applicant: Cognis IP Management GmbH, 40589 Düsseldorf (DE)
(72) Inventor: Kawa, Rolf, 40789 Monheim (DE); Brüning, Stefan, 40593 Düsseldorf (DE)

(57) **Abstract**

The invention is directed to a mixture of esters according to the general formula (1), R₁-C(=O)-Q-R₂. wherein R₁ is an alkyl moiety with 7 to 9 carbon atoms and wherein R₂ is a an alkyl moiety with 8 to 10 carbon atoms, wherein the mixture comprises 5 to 60 weight-% of ester of the general formula (I), wherein R₁ is an alkyl moiety with 9 carbon atoms, based on the total amount of esters according to formula (I) and/or wherein the mixture comprises 5 to 60 weight-% of ester of the general formula (I), wherein R₂ is an alkyl moiety with 10 carbon atoms, based on the total amount of esters according to formula (I). The invention is further directed to cosmetic and/or pharmaceutical compositions comprising such esters as well as to a process for the production of such esters.

## Description

### Field of the Invention

This invention relates to ester mixtures and to cosmetic and/or pharmaceutical compositions comprising such ester mixtures.

### Prior Art

Cosmetic hair and skin-care emulsions are expected by the consumer to satisfy a number of requirements. Apart from the cleansing and caring effects which determine the particular application, importance is attributed to such diverse parameters as highest possible dermatological compatibility, good lipid-layer-enhancing properties, elegant appearance, optimal sensory impression and shelf life.

Besides a number of surfactants, cosmetic hair- and skin-care preparations generally contain, above all, oil components and water. The oil component (emollients) used include, for example, hydrocarbons, ester oils and vegetable and animal oilstfats/waxes. In order to satisfy stringent market requirements in regard to sensory properties and optimal dermatological compatibility, new oil components and emulsifier mixtures are being continuously developed and tested. The use of ester oils in cosmetic products has been known for some time. Volatile silicone oils (cyclopentasiloxane, cyclohexasiloxane) are synthetic substances which evaporate on skin and provide specific properties such as high spreading, anti-tackiness and non greasy skin feel. The use of volatile silicones is widely spread but has been recently under review due to their possible negative effect towards the environment (bioaccumulation) and health. A drawback of known ester oils is their dissatisfactory sensory performance in comparison to silicon oils. One of the goals of the present invention was to provide substances, which can be used as (at least partial) replacement for silicon oils, which provide sensory properties which are comparable to known silicon oils. It was also desirable to provide substances which display better biodegradability and/or less irritation potential, e.g. better skin and/or eye tolerance.

The problem addressed by the present invention was to provide esters, which are preferably liquid at 20°C for cosmetic applications which would have an improved profile in regard to their sensory properties (lightness, non-greasy skin feel, softness, smoothness, spreadability, absorption, distribution behavior, oiliness) and which can be incorporated in a number of cosmetic formulations. Such esters should also be able to serve as a basis for pharmaceutical compositions. The hydrolysis stability of the esters and their capacity for formulation at low pH values would also be of interest in this regard. Furthermore, especially for make-up formulations, the non transfer property is of enhanced interest. In addition the compatibility of the esters with detergent containing compositions (e.g. in shower gel, shampoo, hair conditioner) was of interest.

In addition, the esters should be easily incorporated in both w/o and in o/w formulations and water system like body oil, lipstick AP/Deo stick and should be compatible in particular with crystalline UV filters, pigments, antiperspirants, salts and silicones. It has surprisingly been found that ester mixtures according to claim 1 lead to sensorically light products.

**Cetiol®LC** is an ester mixture commercially available from Cognis GmbH, which is obtained by esterification of a C8 to C10 fatty acid with a saturated fatty alcohol C12 to C18. This ester mixture does not comprise esters according to formula (I), wherein R₂ is an alkyl moiety 8 to 10 carbon atoms. Unfortunately, these esters are problematic in solubilizing crystalline UV filters as well as solubilizing powders or pigment wetting agents. Surprisingly it has been found that the ester mixtures according to the invention displays an improved solubilizing capacity for these agents in comparison to the ester mixture Cetiol®LC. It has furthermore been found, that the ester mixture according to the invention display a higher spreading value in comparison to Cetiol®LC. Accordingly, the problem addressed by the present invention was to provide ester mixtures that would be improved in relation to the prior art, more particularly ester mixtures which could readily be formulated together with UV filters and, at the same time, would not have any disadvantages compared to the prior art in regard to sensory impression (so called "skin feel" or "skin afterfeel").

### Description of the Invention

The present invention relates to mixture of esters according to the general formula (I),

R₁-C(=O)-O-R₂

wherein R₁ is an alkyl moiety with 7 to 9 carbon atoms and wherein R₂ is a an alkyl moiety with 8 to 10 carbon atoms, wherein the mixture comprises 5 to 60 weight-% of ester of the general formula (I), wherein **R₁ is an alkyl moiety with 9 carbon atoms**, based on the total amount of esters according to formula (I) and/or wherein the mixture comprises 5 to 60 weight-% of ester of the general formula (I), wherein **R₂ is an alkyl moiety with 10 carbon atoms**, based on the total amount of esters according to formula (I). The ester mixture according to the invention comprises at least two different esters according to formula (I).

Surprisingly, the ester mixtures (synonymously used are the term "esters" and "mixture of esters") according to the invention are particularly suitable for cosmetic and/or pharmaceutical compositions, more particularly for compositions expected to impart a "light" skin feel. The esters can be incorporated particularly well in various formulations. Liquid substance mixtures are obtained and may be preferably used as oil components or consistency factors. Surprisingly, the ester mixtures according to the invention are particularly suitable for solubilising crystalline UV filters as well as powders or pigment wetting agents. The esters according to the invention are therefore especially suitable as solubilising agents in cosmetic and/or pharmaceutical compositions.

One embodiment of the invention is directed to a mixture of esters according to the general formula (I), R₁-C(=O)-O-R₂ , wherein R₁ is an alkyl moiety with 7 to 9 carbon atoms and wherein R₂ is a an alkyl moiety with 8 to 10 carbon atoms, wherein the mixture comprises 5 to 60 weight-% of ester of the general formula (I), wherein **R₁ is an alkyl moiety with 9 carbon atoms**, based on the total amount of esters according to formula (I).

One embodiment of the invention is directed to a mixture of esters according to the general formula (I), R₁-C(=O)-O-R₂, wherein R₁ is an alkyl moiety with 7 to 9 carbon atoms and wherein R₂ is a an alkyl moiety with 8 to 10 carbon atoms, wherein the mixture comprises 5 to 60 weight-% of ester of the general formula (I), wherein **R₂ is an alkyl moiety with 10 carbon atoms**, based on the total amount of esters according to formula (I).

One embodiment of the invention relates to mixture of esters according to the general formula (I), R₁-C(=O)-O-R₂, wherein R₁ is an alkyl moiety with 7 to 9 carbon atoms and wherein R₂ is a an alkyl moiety with 8 to 10 carbon atoms, wherein the mixture comprises 5 to 60 weight-% of ester of the general formula (I), wherein **R₁ is an alkyl moiety with 9 carbon atoms**, based on the total amount of esters according to formula (I), and wherein the mixture comprises 5 to 60 weight-% of ester of the general formula (I), wherein **R₂ is an alkyl moiety with 10 carbon atoms**, based on the total amount of esters according to formula (I).

An embodiment of the invention is directed to ester mixtures according to the general formula (I), wherein the mixture comprises 5 to 40, preferably 5 to 20 weight-%, more preferably 5 to 10 weight-% of ester of the general formula (I), wherein **R₁ is an alkyl moiety with 9 carbon atoms**, based on the total amount of esters according to formula (I). An embodiment of the invention is directed to ester mixtures according to the general formula (I), wherein the mixture comprises 20 to 40 weight-% of ester of the general formula (I), wherein **R₁ is an alkyl moiety with 9 carbon atoms**, based on the total amount of esters according to formula (I). An embodiment of the invention is directed to ester mixtures according to the general formula (I), wherein the mixture comprises 40 to 60 weight-% of ester of the general formula (I), wherein **R₁ is an alkyl moiety with 9 carbon atoms**, based on the total amount of esters according to formula (I).

An embodiment of the invention is directed to a mixture of esters according to the general formula (I), R₁-C(=O)-O-R₂, wherein R₁ is an alkyl moiety with 7 to 9 carbon atoms and wherein R₂ is a an alkyl moiety with 8 to 10 carbon atoms, wherein the mixture comprises 5 to 40 weight-%, preferably 5 to 20 weight-%, more preferably 5 to 10 weight-% of ester of the general formula (I), wherein **R₂ is an alkyl moiety with 10 carbon atoms**, based on the total amount of esters according to formula (I). An embodiment of the invention is directed to a mixture of esters according to the general formula (I), R₁-C(=O)-O-R₂, wherein R₁ is an alkyl moiety with 7 to 9 carbon atoms and wherein R₂ is a an alkyl moiety with 8 to 10 carbon atoms, wherein the mixture comprises 20 to 40 weight-%, of ester of the general formula (I), wherein **R₂ is an alkyl moiety with 10 carbon atoms**, based on the total amount of esters according to formula (I). An embodiment of the invention is directed to a mixture of esters according to the general formula (I), R₁-C(=O)-O-R₂, wherein R₁ is an alkyl moiety with 7 to 9 carbon atoms and wherein R₂ is a an alkyl moiety with 8 to 10 carbon atoms, wherein the mixture comprises 40 to 60 weight-%, of ester of the general formula (I), wherein **R₂ is an alkyl moiety with 1 carbon atoms**, based on the total amount of esters according to formula (I).

In formula (I) **R₁** is an alkyl moiety with 7 to 9 carbon atoms. Suitable alkyl moieties R₁ are linear or branched, saturated or unsaturated alkyl moieties. Examples of suitable alkyl moieties R₁ are n-heptyl, 1-methylhexyl-, 2-methylhexyl-, 3-methylhexyl-, 4-methylhexyl-, 5-methylhexyl, 1-ethyl-pentyl 1-hepentyl, 2-heptenyl, 3-heptenyl-, 4-heptenyl-, 5-heptenyl, 6-heptenyl-, n-octyl, 2-Ethylhexyl-, 1,1,3,3-Tetramethylbutyl, n-nonyl-, iso-nonyl (preferably 3,5,5 trimethylhexyl-, 2,4,4, ttimethylpentyl-) moieties. In a preferred embodiment of the invention, R₁ is a linear alkyl moiety. In a preferred embodiment of the invention R₁ is a saturated alkyl moiety. Examples of suitable linear and saturated alkyl moieties R₁ are n-heptyl, n-octyl-, and n-nonyl.

In formula (I) **R₂** is an alkyl moiety with 8 to 10 carbon atoms. Suitable alkyl moieties R₂ are linear or branched, saturated or unsaturated alkyl moieties. Examples of suitable alkyl moieties R₂ are n-octyl, 2-ethylhexyl-, 1,1,3,3-tetramethylbutyl, n-nonyl-, n-decyl-, 2-propyl-heptyl, 3-propyl-heptyl, 4-propyl-heptyl, iso-nonyl (preferably 3,5,5 trimethylhexyl or 2,4,4-trimethyl), iso-decyl (preferably 3,5-dimethyl-octyl, trimethyl-heptyl) moieties. In a preferred embodiment of the invention, R₂ is a linear alkyl moiety. In a preferred embodiment of the invention R₂ is a saturated alkyl moiety.

A preferred embodiment of the invention is directed to an ester mixture according to formula (I), wherein the **amount of branched esters** is 50 or less than 50 weight-%, preferably 40 or less than 40 weight-%, is 30 or less than 30 weight-%, is 25 or less than 25 weight-%, is 20 or less than 20 weight-%, is 15 or less than 15 weight %, is 10 or less than 10 weight-%, preferably 5 or less than 5 weight-%, most preferably 1 or less than 1 weight.-%, based on the total amount of esters according to formula (I). A branched ester according to the invention is an ester according to formula (I) wherein R₁ and/or R₂ cany a branched alkyl moiety.

Preferably the ester mixture according to the invention is liquid at 20 °C.

### Production of ester mixtures

The ester mixtures according to the invention can be obtained either by mixing single esters or ester mixtures so that mixtures according to formula (I) are obtained. Alternatively, the ester mixtures according to formula (I) can be obtained by esterifying the respective mixtures of carbon acids with the respective mixtures of alcohols. Likewise the ester mixtures according to formula (I) can be obtained by transesterification of the respective carbon acid methyl ester (mixtures) with the respective alcohol (mixtures).

The present invention also relates to a process for the production of ester mixtures of formula (I), R₁C(=O)-O-R₂ wherein a carbon acid or a mixture of carbon acids R₁-COOH is reacted with an alcohol or a mixture of alcohols R₂-OH, wherein R₁ is an alkyl moiety with 7 to 9 carbon atoms and wherein R₂ is a an alkyl moiety with 8 to 10 carbon atoms, in such a ratio, that the resulting ester mixture comprises 5 to 60 weight-% of ester of the general formula (I), wherein **R₁ is an alkyl moiety with 9 carbon atoms**, based on the total amount of esters according to formula (I) and/or the mixture comprises 5 to 60 weight-% of ester of the general formula (I), wherein **R₂ is an alkyl moiety with 10 carbon atoms**, based on the total amount of esters according to formula (I). This can for example be achieved by reacting the respective amount of carbon acid (or carbon acid mixtures) with the respective amounts of alcohol (or alcohol mixtures) in equimolar amounts.

In a preferred embodiment of the invention, the mixture containing the carbon acid (or carbon acid mixture) and the alcohol (or alcohol mixture) is reacted in the presence of an esterification catalyst.

In a preferred embodiment of the invention, the mixture containing the carbon acid (or carbon acid mixture) and the alcohol (or alcohol mixture) is heated, the water formed is continuously removed and the crude product is then distilled. The process may be carried out in the presence of an esterification catalyst, for example an acid or a base. In a preferred embodiment, the process is carried out in the absence of solvents, preferably with educts which are substantially water-free. A preferred embodiment of the process is characterized by the use of a tin catalyst. Suitable tin catalysts are, for example, tin oxalate (for example Fascat® 2001), tin oxide (SnO, Fascat® 2000) and tin(IV) catalysts, such as dibutyl tin diacetate (Fascat®) 4200), dibutyl tin oxide (Fascat® 4201) and dibutyl tin laurate (Fascat® 4202) or tin oxide (SnO) which were once marketed by Atofina, but are now marketed by Arkema. The esterification is preferably carded out at temperatures in the range from 100 to 300°C and, more particularly, at temperatures in the range from 200 to 250°C.

In another embodiment, at least one enzyme is used as the catalyst. Suitable enzymes are any enzymes or enzyme mixtures known to the expert which are capable of catalyzing the esterification of alcohol and acid, for example lipases, acyl transferases and esterases. The enzyme-catalyzed esterification is typically carried out at temperatures of 20 to 100°C and preferably at temperatures of 40 to 80°C.

The present invention also relates to a process for the production of ester mixtures for formula (I), R₁-C(=O)-O-R₂ wherein a carbon acid alkyl ester or a mixture of carbon acid alkyl esters R₁-C(=O)-R₃ are reacted with an alcohol or a mixture of alcohols R₂-OH, wherein R₁ is an alkyl moiety with 7 to 9 carbon atoms and wherein R₂ is an alkyl moiety with 8 to 10 carbon atoms wherein R₃ is an alkyl moiety with 1, 2, 3 or 4 carbon atoms, in the presence of a transesterification catalyst, in such a ratio, that the resulting ester mixture comprises 5 to 60 weight-% of ester of the general formula (I), wherein R₁ is an alkyl moiety with 9 carbon atoms, based on the total amount of esters according to formula (I) and/or the mixture comprises 5 to 60 weight-% of ester of the general formula (I), wherein R₂ is an alkly moiety with 10 carbon atoms, based on the total amount of esters according to formula (I). In a preferred embodiment R₃ is selected from the group consisting of Methyl-, Ethyl-, n-Butyl.

The present invention also relates to a process for the production of ester mixtures of formula (I), R₁-C(=O)-O-R₂ wherein a carbon acid methyl ester or a mixture of carbon acid methyl esters R₁-C(=O)-CH₃ are reacted with an alcohol or a mixture of alcohols R₂-OH, wherein R₁ is an alkyl moiety with 7 to 9 carbon atoms and wherein R₂ is a an alkyl moiety with 8 to 10 carbon atoms in the presence of a transesterification catalyst, in such a ratio, that the resulting ester mixture comprises 5 to 60 weight-% of ester of the general formula (I), wherein **R₁ is an alkyl moiety with 9 carbon atoms**, based on the total amount of esters according to formula (I) and/or the mixture comprises 5 to 60 weight-% of ester of the general formula (I), **wherein R₂ is an alkyl moiety with 10 carbon atoms**, based on the total amount of esters according to formula (I).

This can for example be achieved by reacting the respective amount of carbon acid alkyl ester (or carbon acid alkyl ester mixtures) with the respective amounts of alcohol (or alcohol mixtures) in equimolar amounts.

In a preferred embodiment, the mixture containing the carbon acid alkyl ester (or carbon acid alkyl ester mixture) and the alcohol (or alcohol mixture) is heated in the presence of the transesterification catalyst, the water formed is continuously removed and the crude product is distilled. In a preferred embodiment, the process is carried out in the absence of solvents, preferably with educts which are substantially water-free. The transesterification is preferably carried out at temperatures of 100 to 300°C and more particularly at temperatures of 200 to 250°C. The transesterification catalyst used may be selected from any of those known to the expert, sodium methylate or tetra-alkyl titanate being preferred.

In another embodiment, at least one enzyme is used as the catalyst. Suitable enzymes are any enzymes or enzyme mixtures known to the expert which are capable of catalyzing the transesterification of alcohol and acid methyl ester, for example lipases, acyl transferases and esterases. The enzyme-catalyzed transesterification is typically carried out at temperatures of 20 to 100°C and preferably at temperatures of 40 to 80°C.

### Cosmetic and/or pharmaceutical compositions

The ester mixture according to the invention can be suitably used in cosmetic and/or pharmaceutical composition. A further embodiment of the invention is therefore directed to a cosmetic and/or pharmaceutical composition comprising 0,1 to 95 weight-% of a mixture of esters according to the general formula (I), R₁-C(=O)-O-R₂, wherein R₁ is an alkyl moiety with 7 to 9 carbon atoms and wherein R₂ is a an alkyl moiety with 8 to 10 carbon atoms,
wherein the mixture comprises 5 to 60 weight-% of ester of the general formula (I), wherein **R₁ is an alkyl moiety with 9 carbon atoms**, based on the total amount of esters according to formula (I) and/or wherein the mixture comprises 5 to 60 weight-% of ester of the general formula (I), wherein **R₂ is an alkyl moiety with 10 carbon atoms**, based on the total amount of esters according to formula (I).

A further embodiment of the invention is therefore directed to the use of an ester mixture according to formula (I) for the preparation of or in cosmetic and/or pharmaceutical compositions, preferably as oil component and/or as solubilizer.

The compositions according to the invention and the ester mixtures according to the invention are suitable for incorporation in all cosmetic preparations such as, for example, body care and cleansing preparations, such as body oil, baby oil, body milk, creams, lotions, sprayable emulsions, sunscreens, antiperspirants, liquid and bar soaps, etc. They may also be used in surfactant-containing formulations such as, for example, foam and shower baths, hair shampoos and hair care rinses, They may be applied as a care component to tissues, papers, wipes, nonwovens, sponges, puffs, plasters and bandages which are used in the field of hygiene and care (wet wipes for baby hygiene and baby care, cleaning wipes, facial wipes, skin care wipes, care wipes containing active ingredients against ageing of the skin, wipes containing sun protection formulations and insect repellents and wipes for decorative cosmetics or for after-sun treatment, toilet wipes, antiperspirant wipes, diapers, handkerchiefs, wet wipes, hygiene products, self-tanning wipes). They may also be used inter alia in hair-care, hair-cleaning or hair-coloring compositions. They may furthermore also be used in decorative cosmetics, such as lipsticks, foundations, make-up, powder, eye-shadow and the like.

A further aspect of the invention is directed to the use of the ester mixtures according to the invention in cosmetic and/or pharmaceutical compositions for moistening or coating of substrates, which are used for cleaning and/or caring of the body and/or hair.

A further embodiment of the invention is directed to **cosmetic and/or pharmaceutical compositions** comprising
(a) 0,1 to 95 weight-% of a mixture of esters according to the general formula (I) R₁C(=O)-O-R₂, wherein R₁ is an alkyl moiety with 7 to 9 carbon atoms and wherein R₂ is a an alkyl moiety with 8 to 10 carbon atoms,
   wherein the mixture comprises 5 to 60 weight-% of ester of the general formula (I), wherein **R₁ is an alkyl moiety with 9 carbon atoms**, based on the total amount of esters according to formula (I) and/or wherein the mixture comprises 5 to 60 weight-% of ester of the general formula (I), wherein **R₂ is an alkyl moiety with 10 carbon atoms**, based on the total amount of esters according to formula (I).
(b) at least one emulsifier (b-1) and/or at least one surfactant **(b-2)** and/or at least one wax component **(b-3)** and/or at least one polymer **(b-4)** and/or at least one other oil component **(b-5).**

The compositions according to the invention preferably contain 0,1 to 80 weight-%, more particularly 0.5 to 70 weight-%, preferably 0.75 to 60 weight-%, more particularly 1 to 50 weight-%, preferably 1 to 40 weight-% of an ester mixture according to formula (I).

The present invention also relates to cosmetic and/or pharmaceutical compositions containing
(a) 0.1 to 95 weight-%, more particularly 0.1 to 80 weight-%, more particularly 0.1 to 70 weight-%, preferably 0.1 to 60 weight-%, more particularly 0.1 to 50 weight-%, preferably 0.1 to 40 weight-% of an ester mixture according to formula (I)
(b) 0.1 to 20 weight-% of emulsifier **(b-1)** and/or **surfactant (b-2)** and/or **wax component (b-3)** and/or **polymer (b-4),** 0.1 to 40% by weight of **other oil components (b-5)** and
(c) 0 to 98% by weight of water.

All percentages by weight represent weight-%, based on the cosmetic and/or pharmaceutical composition, unless otherwise stated.

### Emulsifier b-1

In one embodiment of the invention, the compositions according to the invention contain at least one emulsifier. The compositions according to the invention can contain the emulsifier(s) in a quantity of 0 to 40 weight-%, preferably 0.1 to 20 weight-%, preferably 0.1 to 15 weight-% and more particularly 0.1 to 10 weight-%, based on the total weight of the composition.

In one embodiment of the invention, the composition according to the invention contains more than one emulsifier. Depending on the other components, the expert uses typical emulsifier systems (such as, for example, emulsifier and co-emulsifier).

### Nonionic emulsifiers

The group of nonionic emulsifiers includes, for example,
(1) products of the addition of 2 to 50 mol ethylene oxide and/or 1 to 20 mol propylene oxide onto linear fatty alcohols containing 8 to 40 carbon atoms, onto fatty acids containing 12 to 40 carbon atoms and onto alkylphenols containing 8 to 15 carbon atoms in the alkyl group;
(2) C₁₂₋₁₈ fatty acid monoesters and diesters of products of the addition of 1 to 50 mol ethylene oxide onto glycerol;
(3) sorbitan monoesters and diesters of saturated and unsaturated fatty acids containing 6 to 22 carbon atoms and ethylene oxide adducts thereof;
(4) alkyl mono- and oligoglycosides containing 8 to 22 carbon atoms in the alkyl group and ethoxylated analogs thereof;
(5) products of the addition of 7 to 60 mol ethylene oxide onto castor oil and/or hydrogenated castor oil;
(6) polyol esters and, in particular, polyglycerol esters such as, for example, polyolpofy-12-hydroxystearate, polyglycerol polyricinoleate, polyglycerol diisostearate or polyglycerol dimerate. Mixtures of compounds from several of these classes are also suitable;
(7) products of the addition of 2 to 15 mol ethylene oxide onto castor oil and/or hydrogenated castor oil;
(8) partial esters based on linear, branched, unsaturated or saturated C₆₋₂₂ fatty acids, ricinoleic acid and 12-hydroxystearic acid and polyglycerol, pentaerythritol, dipentaerythritol, sugar alcohols (for example sorbitol), alkyl glucosides (for example methyl glucoside, butyl glucoside, lauryl glucoside) and polyglucosides (for example cellulose) or mixed esters.
(9) polysiloxane/polyalkyl polyether copolymer or corresponding derivatives;
(10) mixed esters of pentaerythritol, fatty acids, citric acid and fatty alcohol and/or mixed esters of fatty acids containing 6 to 22 carbon atoms, methyl glucose and polyols, preferably glycerol or polyglycerol.

The addition products of ethylene oxide and/or propylene oxide onto fatty alcohols, fatty acids, alkylphenols, glycerol monoesters and diesters and sorbitan monoesters and diesters of fatty acids or onto castor oil are known commercially available products. They are homolog mixtures of which the average degree of alkoxylation corresponds to the ratio between the quantities of ethylene oxide and/or propylene oxide and substrate with which the addition reaction is carried out. These emulsifiers are w/o or o/w emulsifiers, depending on the degree of ethoxylation. C_{12/18} fatty acid monoesters and diesters of addition products of ethylene oxide onto glycerol are known as lipid layer enhancers for cosmetic compositions.

According to the invention, particularly suitable and mild emulsifiers are the polyol poly-12-hydroxystearates and mixtures thereof marketed by Cognis Deutschland GmbH under the name of "Dehymuis® PGPH" (w/o emulsifier) or "Eumulgin® VL 75" (mixture with Coco Glucosides in a ratio by weight of 1:1, o/w emulsifier) or "Dehymuls® SBL" (w/o emulsifier). Particular reference is made in this connection to EP 0 766 661 B1**.** The polyol component of these emulsifiers may be derived from substances which contain at least two, preferably 3 to 12 and more particularly 3 to 8 hydroxyl groups and 2 to 12 carbon atoms.

In principle, suitable lipophilic w/o emulsifiers are emulsifiers with an HLB value of 1 to 8 which are listed in numerous Tables and are well-known to the expert. Some of these emulsifiers are listed, for example, in Kirk-Othmer, "Encyclopedia of Chemical Technology", 3rd Edition, 1979, Vol. 8, page 913. The HLB value for ethoxylated products may also be calculated to the following formula: HLB = (100-L) : 5, where L is the percentage by weight of lipophilic groups, i.e. fatty alkyl or fatty acyl groups, in percent by weight in the ethylene oxide adducts.

Of particular advantage from the group of w/o emulsifiers are partial esters of polyols, more particularly C₄₋₆ polyols, such as for example partial esters of pentaerythritol or sugar esters, for example sucrose distearate, sorbitan monoisostearate, sorbitan sesquiisostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 and preferably 5 to 10 mol ethylene oxide onto the sorbitan esters mentioned are also suitable emulsifiers.

Depending on the formulation, it can also be of advantage additionally to use at least one emulsifier from the group of nonionic o/w emulsifiers (HLB value: 8-18) and/or solubilizers. Examples of such emulsifiers are the ethylene oxide adducts mentioned at the beginning with a correspondingly high degree of ethoxylation, for example 10-20 ethylene oxide units for o/w emulsifiers and 20-40 ethylene oxide units for so-called solubilizers. Particularly advantageous o/w emulsifiers for the purposes of the invention are Ceteareth-12 and PEG-20 Stearate. Particularly suitable solubilizers are Eumulgin® HRE 40 (INCI name: PEG-40 Hydrogenated Castor oil), Eumulgin® HRE 60 (INCI name: PEG-60 Hydrogenated Castor Oil), Eumulgin® L (INCI name: PPG-1-PEG-9 Laurylglycolether) and Eumulgin® SML 20 (INCI name: Polysorbat-20).

Nonionic emulsifiers from the group of alkyl oligoglycosides are particularly compatible with the skin. C₈₋₂₂ alkyl mono- and oligoglycosides, their production and their use are known from the prior art. They are produced in particular by reacting glucose or oligosaccharides with primary alcohols containing 8 to 22 carbon atoms. So far as the glycoside component is concerned, both monoglycosides where a cyclic sugar unit is attached to the fatty alcohol by a glycoside bond and oligomeric glycosides with a degree of oligomerization of preferably up to about 8 are suitable. The degree of oligomerization is a statistical mean value on which a homolog distribution typical of such technical products is based. Products available under the name of Plantacare® contain a C₈₋₁₆ alkyl group attached by a glucosidic bond to an oligoglucoside unit with an average degree of oligomerization of 1 to 2. The acyl glucamides derived from glucamine are also suitable nonionic emulsifiers. The product marketed under the name of Emuigade® PL 68/50 by Cognis Deutschland GmbH, which is a 1:1 mixture of alkyl polyglucosides and fatty alcohols, is preferred for the purposes of the invention. According to the invention, the mixture of Lauryl Glucoside, Polyglyceryl-2-Dipolyhydroxystearate, glycerol and water which is marketed as Eumulgin® VL 75 may also be used with advantage in accordance with the invention.

Other suitable emulsifiers are such substances as lecithins and phospholipids. Examples of natural lecithins are the kephalins which are also known as phosphatidic acids and which are derivatives of 1,2-diacyl-sn-glycerol-3-phosphoric acids. By contrast, phospholipids are generally understood to be mono- and preferably diesters of phosphoric acid with glycerol (glycerophosphates) which are generally classed as fats. Sphingosines and sphingolipids are also suitable as fat-like substances.

### Surfactants b-2)

In one embodiment of the invention, the compositions according to the invention contain at least one surfactant The surfactant(s) may be selected from anionic, nonionic, cationic and/or amphoteric or zwitterionic surfactants. Surfactant-containing cosmetic compositions, such as for example shower gels, foam baths, shampoos, etc., preferably contain at least one anionic surfactant.

The compositions according to the invention can contain the surfactant(s) in a quantity of 0 to 40 weight-%, preferably 0 to 20 weight-%, preferably 0.1 to 15 weight-% and more particularly 0.1 to 10 weight-%, based on the total weight of the composition.

Typical examples of **nonionic surfactants** are fatty alcohol polyglycol ethers, alkylphenol polyglycol ethers, fatty acid polyglycol esters, fatty acid amide polyglycol ethers, fatty amine polyglycol ethers, alkoxylated triglycerides, mixed ethers and mixed formals, optionally partly oxidized alk(en)yl oligoglycosides or glucuronic acid derivatives, fatty acid-N-alkyl glucamides, protein hydrolyzates (particularly wheat-based vegetable products), polyol fatty acid esters, sugar esters, sorbitan esters, polysorbates and amine oxides. If the nonionic surfactants contain polyglycol ether chains, they may have a conventional homolog distribution, although they preferably have a narrow-range homolog distribution.

**Zwitterionic surfactants** are surface-active compounds which contain at least one quaternary ammonium group and at least one -COO^{{-}} or -SO₃^{{-}} group in the molecule. Particularly suitable zwitterionic surfactants are the so-called betaines, such as the N-alkyl-N,N-dimethyl ammonium glycinates, for example cocoalkyl dimethyl ammonium glycinate, N-acylaminopmpyl-N,N-dimethyl ammonium glycinates, for example cocoacylaminopropyl dimethyl ammonium glycinate, and 2-alkyl-3-carboxymethyl-3-hydroxyethyl imidazolines containing 8 to 18 carbon atoms in the alkyl or acyl group and cocoacylaminoethyl hydroxyethyl carboxymethyl glycinate. The fatty acid amide derivative known under the CTFA name of *Cocamidopropyl Betaine* is particularly preferred. Ampholytic surfactants are also suitable, particularly as co-surfactants. Ampholytic surfactants are surface-active compounds which, in addition to a C₈₋₁₈ alkyl or acyl group, contain at least one free amino group and at least one -COOH- or -SO₃H- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkyl-aminopropionic acids and alkylaminoacetic acids containing around 8 to 18 carbon atoms in the alkyl group. Particularly preferred ampholytic surfactants are N-cocoalkylaminopropionate, cocoacylaminoethyl aminopropionate and C_{12/18} acyl sarcosine.

**Anionic surfactants** are characterized by a water-solubilizing anionic group such as, for example, a carboxylate, sulfate, sulfonate or phosphate group and a lipophilic group. Dermatologically safe anionic surfactants are known to the expert in large numbers from relevant textbooks and are commercially available. They are, in particular, alkyl sulfates in the form of their alkali metal, ammonium or alkanolammonium salts, alkylether sulfates, alkylether carboxylates, acyl isethionates, acyl sarcosinates, acyl taurines containing linear C₁₂₋₁₈ alkyl or acyl groups and sulfosuccinates and acyl glutamates in the form of their alkali metal or ammonium salts. Particularly suitable anionic surfactants are Glyceryl stearate citrates or glyceryl stearate lactates.

Particularly suitable **cationic surfactants** are quaternary ammonium compounds, preferably ammonium halides, more especially chlorides and bromides, such as alkyl trimethyl ammonium chlorides, dialkyl dimethyl ammonium chlorides and trialkyl methyl ammonium chlorides, for example cetyl trimethyl ammonium chloride, stearyl trimethyl ammonium chloride, distearyl dimethyl ammonium chloride, lauryl dimethyl ammonium chloride, lauryl dimethyl benzyl ammonium chloride and tricetyl methyl ammonium chloride. In addition, the readily biodegradable quaternary ester compounds, such as for example the dialkyl ammonium methosulfates and methyl hydroxyalkyl dialkoyloxyalkyl ammonium methosulfates marketed under the name of Stepantex® and the corresponding products of the Dehyquart® series, may be used as cationic surfactants. "Esterquats" are generally understood to be quatemized fatty acid triethanolamine ester salts. They can provide the compositions with particular softness. They are known substances which are prepared by the relevant methods of organic chemistry. Other cationic surfactants suitable for use in accordance with the invention are the quatemized protein hydrolyzates.

### Wax component b-3)

In one embodiment of the invention, the preparations according to the invention contain at least one wax component. The compositions according to the invention can contain the wax component(s) in a quantity of 0 to 40 weight-%, more particularly 0 to 20 weight-%, preferably 0.1 to 15 weight-% and more particularly 0.1 to 10 weight-%, based on the total weight of the composition.

Waxes are normally understood to be natural or synthetic substances and mixtures having the following properties: they have a solid to brittle hard consistency, are coarsely to finely crystalline, transparent to opaque and melt above 30°C without decomposing. Even slightly above their melting point, they are low in viscosity and non-stringing and are very temperature-dependent in their consistency and solubility. A single wax component or a mixture of wax components melting at or above 30°C may be used in accordance with the invention. According to the invention, fats and fat-like substances with a wax-like consistency may also be used as waxes providing they have the required melting point These include inter alia fats (triglycerides), mono- and diglycerides, natural and synthetic waxes, fat and wax alcohols, fatty acids, esters of fatty alcohols and fatty acids and fatty acid amides or mixtures of these substances. Fats in the context of the invention are understood to be triacylglycerols, i.e. the triple esters of fatty acids with glycerol. The triacylglycerols preferably contain saturated, unbranched and unsubstituted fatty acid components. They may also be mixed esters, i.e. triple esters of glycerol with various fatty acids. So-called hardened fats and oils obtained by partial hydrogenation may be used in accordance with the invention and are particularly suitable as consistency factors. Vegetable hardened fats and oils, for example hardened castor oil, peanut oil, soybean oil, colza oil, rapeseed oil, cottonseed oil, soybean oil, sunflower oil, palm oil, palm kernel oil, linseed oil, almond oil, corn oil, olive oil, sesame oil, cocoa butter and coconut fat, are preferred. Suitable fats are inter alia the triple esters of glycerol with C₁₂₋₆₀ fatty acids and in particular C₁₂₋₃₆ fatty acids. These include hydrogenated castor oil, a triple ester of glycerol and a hydroxystearic acid which is marketed, for example, under the name of Cutina® HR. Glycerol tristearate, glycerol tribehenate (for example Syncrowax® HRC), glycerol tripalmitate or the triglyceride mixtures known under the name of Syncrowax® HGLC are also suitable providing the melting point of the wax component or the mixture is 30°C or higher. According to the invention, suitable wax components are, in particular, mono- and diglycerides and mixtures of these partial glycerides. Glyceride mixtures suitable for use in accordance with the invention include the products Novata® AB and Novata® B (mixture of C₁₂₋₁₈ mono-, di- and triglycerides) and Cutina® MD or Cutina® GMS (glyceryl stearate) marketed by Cognis GmbH.

The fatty alcohols suitable for use as a wax component in accordance with the invention include C₁₂₋₅₀ fatty alcohols. The fatty alcohols may be obtained from natural fats, oils and waxes such as, for example, myristyl alcohol, 1-pentadecanol, cetyl alcohol, 1-heptadecanol, stearyl alcohol, 1-nonadecanol, arachidyl alcohol, 1-heneicosanol, behenyl alcohol, brassidyl alcohol, lignoceryl alcohol, ceryl alcohol or myricyl alcohol. According to the invention, saturated unbranched fatty alcohols are preferred. However, unsaturated, branched or unbranched fatty alcohols may also be used as the wax component in accordance with the invention providing they have the required melting point Other suitable fatty alcohols are the fatty alcohol cuts obtained in the reduction of naturally occurring fats and oils such as, for example, bovine tallow, peanut oil, colza oil, cottonseed oil, soybean oil, sunflower oil, palm kernel oil, linseed oil, castor oil, com oil, rapeseed oil, sesame oil, cocoa butter and coconut oil, However, synthetic alcohols, for example the linear, even-numbered fatty alcohols from Ziegler's synthesis (Alfols) or the partly branched alcohols from the oxosynthesis (Dobanols) may also be used. C₁₄₋₂₂ fatty alcohols marketed for example by Cognis Deutschland GmbH under the name of Lanette® 16 (C₁₆ alcohol), Lanette® 14 (C₁₄ alcohol), Lanefte® O (C_{16/18} alcohol) and Lanette® 22 (C_{18/22} alcohol) are particularly suitable for the purposes of the invention. Fatty alcohols give the compositions a dryer feeling on the skin than triglycerides.

C₁₄₋₄₀ fatty acids or mixtures thereof may also be used as wax components. These include, for example, myristic, pentadecanoic, palmitic, margaric, stearic, nonadecanoic, arachic, behenic, lignoceric, cerotic, melissic, erucic and elaeostearic acid and substituted fatty acids such as, for example, 12-hydroxystearic acid, and the amides or monoethanolamides of the fatty acids. This list is meant to be purely exemplary without any limiting character.

Waxes suitable for use In accordance with the invention are, for example, natural vegetable waxes, such as candelilla wax, carnauba wax, Japan wax, espartograss wax, cork wax, guaruma wax, rice oil wax, sugar cane wax, ouricury wax, montan wax, sunflower wax, fruit waxes, such as orange waxes, lemon waxes, grapefruit wax, bayberry wax, and animal waxes such as, for example, beeswax, shellac wax, spermaceti, wool wax and uropygial fat. According to the invention, it can be of advantage to use hydrogenated or hardened waxes. Natural waxes usable in accordance with the invention also include the mineral waxes, such as ceresine and ozocerite for example, or the petrochemical waxes, for example petrolatum, paraffin waxes and microwaxes. Other suitable wax components are chemically modified waxes, more particularly the hard waxes such as, for example, montan ester waxes, sasol waxes and hydrogenated jojoba waxes. Synthetic waxes usable in accordance with the invention include, for example, wax-like polyalkylene waxes and polyethylene glycol waxes. Vegetable waxes are preferred for the purposes of the invention.

The wax component may also be selected from the group of wax esters of saturated and/or unsaturated, branched and/or unbranched alkanecarboxylic acids and saturated and/or unsaturated, branched and/or unbranched alcohols, from the group of esters of aromatic carboxylic acids, dicarboxylic acids, tricarboxylic acids and hydroxycarboxylic acids (for example 12-hydroxystearic acid) and saturated and/or unsaturated, branched and/or unbranched alcohols and also from the group of lactides of long-chain hydroxycarboxylic acids. Examples of esters such as these include, for example, C₁₆₋₄₀ alkyl stearates, C₂₀₋₄₀ alkyl stearates (for example Kesterwachs® K82H), C₂₀₋₄₀ dialkyl esters of dimer acids, C₁₈₋₃₈ alkyl hydroxystearoyl stearates or C₂₀₋₄₀ alkyl erucates. Other suitable wax components which may be used are C₃₀₋₅₀ alkyl beeswax, tristearyl citrate, triisostearyl citrate, stearyl heptanoate, stearyl octanoate, trilauryl citrate, ethylene glycol dipalmitate, ethylene glycol distearate, ethylene glycol di(12-hydroxystearate), stearyl stearate, palmityl stearate, stearyl behenate, cetyl ester, cetearyl behenate and behenyl behenate.

### Polymers b-4)

In one embodiment of the invention, the compositions according to the invention contain at least one polymer. The compositions according to the invention can contain the polymer(s) in a quantity of 0 to 20 weight-%, preferably 0.1 to 15 weight-%, and, more particularly, 0.1 to 10 weight-%, based on the total weight of the composition.

Suitable **cationic polymers** are, for example, cationic cellulose derivatives such as, for example, the quaternized hydroxyethyl cellulose obtainable from Amerchol under the name of Polymer JR 400®, cationic starch, copolymers of diallyl ammonium salts and acrylamides, quatemized vinyl pyrrolidone/vinyl imidazole polymers such as, for example, Luviquat® (BASF), condensation products of polyglycols and amines, quatemized collagen polypeptides such as, for example, Lauryldimonium Hydroxypropyl Hydrolyzed Collagen (Lamequat® L, Grünau), quatemized wheat polypeptides, polyethyleneimine, cationic silicone polymers such as, for example, amodimethicone, copolymers of adipic acid and dimethylaminohydroxypropyl diethylenetriamine (Cartaretine®, Sandoz), copolymers of acrylic acid with dimethyl diallyl ammonium chloride (Merquat® 550, Chemviron), polyaminopoly-amides, cationic chitin derivatives such as, for example, quatemized chitosan, optionally in microcrystalline distribution, condensation products of dihaloalkyls, for example dibromobutane, with bis-dialkylamines, for example bis-dimethylamino-1,3-propane, cationic guar gum such as, for example, Jaguar®CBS, Jaguar®C-17, Jaguar®C-16 of Celanese, quatemized ammonium salt polymers such as, for example, Mirapol®A-15, Mirapol® AD-1, Mirapol® AZ-1 of Miranol.

Suitable **anionic, zwitterionic, amphoteric and nonionic polymers are**, for example, vinyl acetate/crotonic acid copolymers, vinyl pyrrolidone/vinyl acrylate copolymers, vinyl acetate/butyl maleate/isobomyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/teft.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized cellulose ethers and silicones. Other suitable polymers are polysaccharides, more particularly xanthan gum, guar guar, agar agar, alginates and tyloses.

### Other oil components b-5)

The cosmetic compositions according to the invention can contain - in addition to the ester mixture according to formula (I) other oil components. The total oil components (esters according to the invention plus other oil components) are typically present in a total quantity of 0.1 to 95, preferably of 0.1 to 80, more particularly 0.5 to 70, preferably 1 to 60, more particularly 1 to 50 weight-%, more particularly 1 to 40 weight-%, preferably 5 to 25 weight-% and more particularly 5 to 15 weight-%. The other oil components are typically present in a quantity of 0.1 to 40 weight-%.

The other oil components may be selected, for example, from Guerbet alcohols based on fatty alcohols containing 6 to 18 and preferably 8 to 10 carbon atoms and other additional esters, such as myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of C₁₈₋₃₈ alkylhydroxycarboxylic acids with linear or branched C₆₋₂₂ fatty alcohols, more especially Dioctyl Malate, esters of linear and/or branched fatty acids with polyhydric alcohols (for example propylene glycol, dimer diol or trimer triol), triglycerides based on C₆₋₁₀ fatty acids, liquid mono-, di-and triglyceride mixtures based on C₆₋₁₈ fatty acids, esters of C₆₋₂₂ fatty alcohols and/or Guerbet alcohols with aromatic carboxylic acids, more particularly benzoic acid, esters of C₂₋₁₂ dicarboxylic acids with polyols containing 2 to 10 carbon atoms and 2 to 6 hydroxyl groups, vegetable oils, branched primary alcohols, substituted cyclohexanes, linear and branched C₆-₂₂ fatty alcohol carbonates such as, for example, Dicaprylyl Carbonate (Cetiol® CC), Guerbet carbonates based on fatty alcohols containing 6 to 18 and preferably 8 to 10 carbon atoms, esters of benzoic acid with linear and/or branched C₆₋₂₂ alcohols (for example Finsolv® TN), linear or branched, symmetrical or nonsymmetrical dialkyl ethers containing 6 to 22 carbon atoms per alkyl group such as, for example, Dicaprylyl Ether (Cetiol® OE), ring opening products of epoxidized fatty acid esters with polyols and hydrocarbons or mixtures thereof (Cetiol® DD).

A further embodiment of the present invention is directed to cosmetic and/or pharmaceutical compositions containing
(a) 0.1 to 95 weight-%, more particularly 0.1 to 80 weight-%, more particularly 0.1 to 70 weight-%, preferably 0.1 to 60 weight-%, more particularly 0.1 to 50 weight-%, preferably 0.1 to 40 weight-% of an ester mixture according to formula (I)
(b) at least **one UV protective factor.**

### UV Protective factor

UV photoprotective factors are, for example, to be understood as meaning organic substances (photoprotective filters) which are liquid or crystalline at room temperature and which are able to absorb ultraviolet rays and give off the absorbed energy again in the form of longer-wavelength radiation, e.g. heat. UVB filters can be oil-soluble or water soluble. Examples of oil-soluble substances are:
- 3-benzylidenecamphor (Mexoryl®SD) or 3-benzylidenenorcamphor (Mexoryl®SDS 20) and derivatives thereof, e.g. 3-(4-methylbenzylidene)camphor
- 3-(4'-Trimethylammonium) benzyliden- boman-2-on-methylsulfat (Mexoryl® SO)
- 3,3'-(1,4-Phenylendimethin)-bis (7,7- dimethyl-2-oxobicyclo-[2.2.1] heptan-1-methansulfonsaure) and salts (Mexaryl®SX)
- 3-(4'-Sulfo)-benzyliden-boman-2-on and salts (Mexoryl®SL)
- Polymer of N-{(2und 4)- [2-oxobom-3-yliden)methyl}benzyl]acrylamid (Mexoryl® SW)
- 2-(2H-Benzotriazol-2-yl)-4-methyl-6-(2-methyl-3-(1,3,3,3-tetramethyl-1-(trimethylsilyloxy) disiloxanyl)propyl) phenol (Mexoryl®XL)
- 4-aminobenzoic acid derivatives, preferably 2-ethylhexyl 4-(dimethylamino)benzoate, 2-octyl 4-(di-methylamino)benzoate and amyl 4-(dimethylamino)benzoate;
- esters of cinnamic acid, preferably 2-ethylhexyl 4-methoxycinnamate, propyl 4-methoxycinnamate, isoamyl 4-methoxycinnamate, 2-ethylhexyl 2-cyano-3,3-phenylcinnamate (octocrylene);
- esters of salicylic acid, preferably 2-ethylhexyl salicylate, 4-isopropylbenzyl salicylate, homomenthyl salicylate;
- derivatives of benzophenone, preferably 2-hydroxy-4-methoxybenzophenone, 2-hydroxy-4-methoxy-4'-methylbenzophenone, 2,2'-dihydroxy-4-methoxybenzo-phenone;
- esters of benzalmalonic acid, preferably di-2-ethylhexyl 4-methoxybenzalmalonate;
- triazine derivatives, such as, for example, 2,4,6-trianilino(p-carbo-2'-ethyl-1'-hexy!oxy)-1,3,5-tnazine, or 2,4,6-Tris[p-(2-ethylhexyl-oxycar-bonyl) anilino]-1,3,5-triazin (Uvinul® T 150) or octyltriazone or *(Uvasorb®HEB);* or diethy[hexyl butamido triazone (Uvasorb^{®} HEB; =4,4'-[(6-[4-((1,1-Dimethylethyl)amino-carbonyl) phenyl-amino]-1,3,5-triazin-2,4- diyl)diimino] bis(benzoesäure-2- ethylhexylester)
- 2,2(-Methylen-bis(6-(2H-benzotriazol-2-yl)-4-(1,1,3,3-tetramethyl-butyl)phenol) (Tinosorb®M);
- 2,4-Bis[4-(2-ethylhexyloxy)-2-hydroxyphenyl]-6-(4-methoxyphenyl)-1,3,5-triazin (Tinosorb® S);
- propane-1,3-diones, such as, for example, 1-(4-tert-butylphenyl)-3-(4'-methoxyphenyl)propane-1,3-dione;
- ketotricyclo(5.2.1.0)decane derivatives, as disclosed in EP 0694521 B1.

Suitable water-soluble substances are:
- 2-phenylbenzimidazole-5-sulphonic acid and the alkali metal, alkaline earth metal, ammonium, alkylammonium, alkanolammonium and glucammonium salts thereof;
- 2,2(-(1,4-Phenylen)bis(1H-benzimidazol-4,6-disulfonic acid, monosodiumsalt) (Neo Heliopan®AP) (INCI: Disodium Phenyl Dibenzimidazole Tetrasulfonate)
- sulphonic acid derivatives of benzophenones, preferably 2-hydroxy-4-methoxybenzophenone-5-sulphonic acid and its salts;
- sulphonic acid derivatives of 3-benzylidenecamphor, such as, for example, 4-(2-oxo-3-bomylidenemethyl)-benzenesulphonic acid and 2-methyl-5-(2-oxo-3-bomylidene)sulphonic acid and salts thereof.

In a preferred embodiment of the invention the compositions comprise at least one oil-soluble UV protective factor and at least one water-soluble UV protective factor.

Suitable typical UV-A filters are, in particular, derivatives of benzoylmethane, such as, for example, 1-(4'-tert-butylphenyl)-3-(4'-methoxyphenyl)propane-1,3-dione, 4-tert-butyl-4'-methoxydibenzoyl-methane (Parsol® 1789), 1-phenyl-3-(4'-isopropylphenyl)propane-1,3-dione, and enamine compounds, as disclosed in DE 19712033 A1 (BASF) as well as Benzoic Acid, 2-[4-(Diethylamino)-2-Hydroxybenzoyl]-, Hexyl Ester (Uvinul® A plus, INCI: Diethylamino hydroxybenzoyl hexyl benzoate. The UV-A and UV-B filters can of course also be used in mixtures. Particularly favourable combinations consist of the derivatives of benzoylmethane, e.g. 4-tert-butyl-4'-methoxydibenzoylmethane (Parsol^{®} 9789) and 2-ethylhexyl 2-cyano-3,3-phenylcinnamate (octocrylene) in combination with esters of cinnamic acid, preferably 2-ethylhexyl 4-methoxycinnamate and/or propyl 4-methoxycinnamate and/or isoamyl 4-methoxycinnamate. Advantageously, such combinations are combined with water-soluble filters such as, for example, 2-phenylbenzimidazole-5-sulphonic acid and their alkali metal, alkaline earth metal, ammonium, alkylammonium, alkanolammonium and glucammonium salts.

Suitable UV-photoprotective factors are especially the ones listed in Annex VII of the Commisiions Directive (in the Version Commission **Directive 2005/9/EC of 28 January 2005 amending Council Directive 761768/EEC,** concerning cosmetic products, for the purposes of adapting Annexes VII thereof to technical progress), which are hereby explicitely referred to.

As well as said soluble substances, insoluble light protection pigments, namely finely dispersed metal oxides or salts, are also suitable for this purpose. Examples of suitable metal oxides are, in particular, zinc oxide and titanium dioxide and also oxides of iron, zirconium, silicon, manganese, aluminium and cerium, and mixtures thereof. Salts which may be used are silicates (talc), barium sulphate or zinc stearate. The oxides and salts are used in the form of the pigments for skincare and skin-protective emulsions and decorative cosmetics. The particles here should have an average diameter of less than 100 nm, preferably between 5 and 50 nm and in particular between 15 and 30 nm. They can have a spherical shape, but it is also possible to use particles which have an ellipsoidal shape or a shape deviating in some other way from the spherical form. The pigments can also be surface-treated, i.e. hydrophilicized or hydrophobicized. Typical examples are coated titanium dioxides, such as, for example, titanium dioxide T 805 (Degussa) or Eusolex^{®} T2000 Eusolex® T-Aqua, Eusolex® AVO, Eusolex® T-ECO, Eusolex® T-OLEO und Eusolex® T-S (Merck). Typical examples of zinc oxides are for example Zinc Oxide neutral, Zinc Oxide NDM (Symrise) or Z-Cote® (BASF) or SUNZnO-AS as well as SUNZnO-NAS (Sunjun Chemical Co. Ltd.). Suitable hydrophobic coating agents are here primarily silicones and, specifically in this case, trialkoxyoctylsilanes or simethicones. In sunscreens, preference is given to using so-called micro- or nanopigments. Preference is given to using micronized zinc oxide.

Further suitable UV protective factors listed in the review of P.Finkel in SÖFW-Journal 122, 8/1996, p. 543 to 548 as well as in Parf.Kosm, 80. Jahrgang, Nr. 3/1999,p.10 to 16 are hereby included by reference.

Besides the two groups of primary UV protection factors mentioned above, secondary UV protection factors of the antioxidant type may also be used. Secondary UV protection factors of the antioxidant type interrupt the photochemical reaction chain which is initiated when UV rays penetrate into the skin.

In a preferred embodiment of the invention, the compositions comprise at least one UV protective factor selected from the group consisting of
- 4-Methylbenzylidene Camphor (Tradename: NeoHeliopan®MBC, supplier: Symrise);
- Benzophenone-3 (Tradename: NeoHeliopan® BB, supplier: Symrise);
- Butyl Methoxydibenzoylmethane (Tradename: Parsol®1789, Hoffmann-La Roche
- Bis-Ethylhexyloxyphenol Methoxyphenyl Triazine (Tradename: Tinosorb®S, CIBA),
- Methylene Bis-Benzotriazolyl Tetramethylbutylphenol (Tradename: Tinosorb®M, supplier: Ciba Specialty Chemicals Corporation;
- Diethylhexyl Butamido Triazone (Tradename: Uvasorb®HEB, supplier 3V Inc.;
- Ethylhexyl Triazone (Tradename: Uvinul®T 150, supplier: BASF AG;
- Diethylamino Hydroxybenzoyl Hexyl Benzoate (Tradename: Uvinul® A plus, BASF SE;
- 3-(4'-Trimethylammonium) benzyliden-boman-2-on-methylsulfat (Tradename: Mexoryl® SO; INCI Camphor Benzalkonium Methosulfate)
- 3,3'-(1,4-Phenylendimethin)-bis(7,7-dimethyl-2-oxobicyclo-[2.2.1]heptan-1-methansulfonic acid (Mexoryl®SX, INCI Terephthalylidene Dicamphor Sulfonic Acid),
- 3-(4'-Sulfo)-benzyliden-boman-2-on, (Mexory®SL; BenzylideneCamphorSulfonicAcid)
- Polymer of N-{(2and 4)-[2-oxobom-3-yliden)methyl)benzyllacrylamid (Tradename: Mexoryl®SW, INCI Polyacrylamidomethyl Benzylidene Camphor)
- 2-(2H-Benzotriazol-2-yl)-4-methyl-6-(2-methyl-3-(1,3,3,3-tetramethyl-1-(trimethyisilyloxy) disiloxanyl)propyl) phenol (INCI: Drometrizole Trisiloxane) and
- Dimethicodiethylbenzalmalonate (Tradename: Parsol® SLX, INCI Polysilicone-15).

The compositions according to the invention can comprise the UV photoprotective factors in an amount of 0,1 to 30 weight-%, preferably 2,5 to 20 Gew.-%, more preferably 5 - 15 weight-%, based on the cosmetic and/or pharmaceutical composition.

### Auxiliaries and additivies

Depending on the application envisaged, the cosmetic compositions contain a number of other auxiliaries and additives such as, for example, consistency factors, thickeners, superfatting agents, stabilizers, polymers, , phospholipids, biogenic agents, antioxidants, deodorants, antiperspirants, antidandruff agents, film formers, swelling agents, insect repellents, self-tanning agents, tyrosinase inhibitors (depigmenting agents), hydrotropes, solubilizers, preservatives, perfume oils, dyes, etc. which are listed by way of example in the following.

Suitable thickeners are, for example, Aerosil® types (hydrophilic silicas), carboxymethyl cellulose and hydroxyethyl and hydroxypropyl cellulose, polyvinyl alcohol, polyvinyl pyrrolidone and bentonites, for example Bentone® Gel VS-5PC (Rheox). In the context of the invention, biogenic agents are, for example, tocopherol, tocopherol acetate, tocopherol palmitate, ascorbic acid, (deaxy)ribonucleic acid and fragmentation products thereof, β-glucans, retinol, bisabolol, allantoin, phytantriol, panthenol, AHA acids, amino acids, ceramides, pseudoceramides, essential oils, plant extracts, for example prunus extract, bambara nut extract, and vitamin complexes. Deodorizing components counteract, mask or eliminate body odors. Body odors are formed through the action of skin bacteria on apocrine perspiration which results in the formation of unpleasant-smelling degradation products. Accordingly, suitable deodorizing components are inter alia germ inhibitors, enzyme inhibitors, odor absorbers or odor maskers. Suitable insect repellents are N,N-diethyl-m-toluamide, pentane-1,2-diol or 3-(N-n-butyl-N-acetylamino)-propionic acid ethyl ester), which is marketed as Insect Repellent 3535 by Merck KGaA, and Butylacetylaminopropionate. A suitable self-tanning agent is, for example, dihydroxyacetone. Suitable tyrosine inhibitors, which prevent the formation of melanin and are used in depigmenting agents, are, for example, arbutin, ferulic acid, koji acid, coumaric acid and ascorbic acid (vitamin C). Suitable preservatives are, for example, phenoxyethanol, formaldehyde solution, parabens, pentanediol or sorbic acid and the silver complexes known under the name of Sunfacine® and the other classes of compounds listed in Appendix 6, Parts A and B of the Kosmetikverordnung ("Cosmetics Directive"). Suitable perfume oils are mixtures of natural and synthetic perfumes. Natural perfumes are extracts of flowers, stems and leaves, fruits, fruit peel, roots, woods, herbs and grasses, needles and branches, resins and balsams. Also suitable are animal raw materials, for example civet and beaver, and synthetic perfume compounds of the ester, ether, aldehyde, ketone, alcohol and hydrocarbon type. Suitable **pearlizing waxes**, particularly for use in surfactant-containing formulations, are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxysubstituted carboxylic acids with fatty alcohols containing 6 to 22 carbon atoms, especially long-chain esters of tartaric acid; fatty compounds, such as for example fatty alcohols, fatty ketones, fatty aldehydes, fatty ethers and fatty carbonates which contain in all at least 24 carbon atoms, especially laurone and distearylether; fatty acids, such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups and mixtures thereof. **Superfatting agents** may be selected from such substances as, for example, lanolin and lecithin and also polyethoxylated or acylated lanolin and lecithin derivatives, polyol fatty acid esters, monoglycerides and fatty acid alkanolamides, the fatty acid alkanolamides also serving as foam stabilizers. Metal salts of fatty acids such as, for example, magnesium, aluminium and/or zinc stearate or ricinoleate may be used as stabilizers. In addition, hydrotropes, for example ethanol, isopropyl alcohol or polyols, may be used to improve flow behavior. Suitable polyols preferably contain 2 to 15 carbon atoms and at least two hydroxyl groups. The polyols may contain other functional groups, more especially amino groups, or may be modified with nitrogen.

The compositions according to the invention and the esters according to the invention are suitable, particularly in cosmetic and/or pharmaceutical compositions, for wetting or impregnating or coating utility and hygiene wipes which are used for care and/or cleansing of the body. Examples of utility and hygiene wipes include tissues, papers, wipes, nonwovens, sponges, puffs, plasters and bandages which are used in the field of hygiene and care. These may be wet wipes for baby hygiene and baby care, cleaning wipes, facial wipes, skin care wipes, care wipes containing active ingredients against ageing of the skin, wipes containing sun protection formulations and insect repellents and wipes for decorative cosmetics or for after-sun treatment, toilet wipes, antiperspirant wipes, diapers, handkerchiefs, wet wipes, hygiene products and self-tanning wipes.

### Examples

### Example 1:

1 mol of a mixture of n-octanoic acid and n-decanoic acid (60 weight-% of n-octanoic acid and 40 weight-% of decanoic acid) and 1,1 mol n-octanol as well as 0,22g Fascat®2001 (Sn oxalat) are combined and heated for 3 h at a temperature of 240°C on a water separator. Then the product is distilled over a 30 cm column (153-168 °C at 0,8 mbar). The product is a colourless and odourless oil. It comprises 40 weight-% of an ester according to the general formula (1), wherein R₁ is a linear and saturated alkyl moiety with 9 carbon atoms.

### Example 2:

1 mol of a mixture of n-octanoic acid and n-decanoic acid (80 weight-% of n-octanoic acid and 20 weight-% of decanoic acid),1,1 mol n-octanol as well as 0,22gFascat®2001 (Sn oxalat) are combined and heated for 3 h at a temperature of 240°C in a water separator. Then the product is distilled over a 30 cm column (153-168 °C at 0,8 mbar). The product is a colourless and odourless oil. It comprises 20 weight-% of an ester according to the general formula (I), wherein R₁ is a linear and saturated alkyl moiety with 9 carbon atoms.

### Example 3:

1 mol of a mixture of n-octanoic acid methyl ester and n-decanoic acid methyl ester (60 weight-% of n-octanoic acid methyl ester and 40 weight-% of decanoic acid methyl ester), 1,1 mol n-octanol as well as 0,22gFascat®2001 (Sn oxalat) are combined and heated for 3 h at a temperature in the range of 180 to 220°C on a water separator. Then the product is distilled over a 30 cm column (153-168°C at 0,8 mbar). The product is a colourless and odourless oil. It comprises 40 weight-% of an ester according to the general formula (I), wherein R₁ is a linear and saturated alkyl moiety with 9 carbon atoms.

### Example 4:

1 mol of n-octanoic acid and 1,1 mol of a mixture of n-octanol and n-decanol (60 weight-% of n-octanol and 40 weight-% of n-decanol) as well as 0,22g Fascat®2001 (Sn oxalat) are combined and heated for 3 h at a temperature of 240 °C on a water separator. Then the product is distilled over a 30 cm column (153-168 °C at 0,8 mbar). The product is a colourless and odourless oil. It comprises 40 weight-% of an ester according to the general formula (1), wherein R₂ is a linear and saturated alkyl moiety with 10 carbon atoms.

In the following compositions, all numbers are weight.-% based on the final compositions.

**Table 1: O/W Body Care Emulsions**

| **Ingredients** | **1** | **2** | **3** | **4** | **5** | **6** | **7** | **8** | **9** | **10** | **11** |
|---|---|---|---|---|---|---|---|---|---|---|---|
| **C - Cream, L - Lotion** | **C** | **C** | **C** | **L** | **C** | **L** | **L** | **C** | **L** | **C** | **C** |
| Eumulgin^{®} VL 75 | | | | | | | | 2,0 | | 1.5 | |
| Dehymuls^{®} PGPH | | 0.6 | | | | | | | | | |
| Generol^{®} R | | | 0.5 | | | | | | | | |
| Eumulgin^{®} B2 | | | 2,0 | | | | | | 2,0 | | |
| Tween^{®} 60 | | | | 0.2 | | | | | | | |
| Cutina^{®} E 24 | | | | 0.2 | | | | | | | |
| Hostaphat^{®} KL 340 N | | | | | | | | | 0.5 | | |
| Lanette^{®} E | | | | | | | | 0.6 | | | |
| Amphisol^{®} K | | | 0.2 | | | | | | | | |
| Sodium Stearate | | | | | 0.5 | | | | | | |
| Emufgade^{®} PL 68/50 | 3,0 | | | | | | 2,0 | | | | 1.2 |
| Eumulgin^{®} SG | 0,2 | | | | 0,2 | 0,3 | | | | | |
| Eumulgin^{®} Prisma | | 0,2 | | | | | 0,2 | | | 0,2 | 0,5 |
| Inwitor 372 P | | | | | | 3,0 | | | | 3,0 | |
| Tego^{®} Care CG | 0.7 | | | | | | | | | | |
| Tego^{®} Care 450 | | | | | 3 | | 1,0 | | | 1,0 | |
| Cutina^{®} PES | 2.5 | 2 | 3 | | | 2 | | 1.7 | 2.5 | | 1.2 |
| Cutina^{®} MD | | 1 | | 3 | 5 | | 2 | | | 3 | |
| Lanette^{®} 14 | | | | 1 | | | | 4 | | | 4 |
| Lanette^{®} O | 4.5 | | 4 | | 1 | | | | | | 2 |
| Novata^{®} AB | | 1 | | | | | | | | | 1 |
| Emery^{®} 1780 | | | | | 0.5 | 0.5 | | | | | |
| Lanolin, water-free, USP | | | | | | | 1.1 | | | | |
| Cosmedia^{®} DC | | 1.5 | 2 | | | 1.5 | 2 | | 1.5 | 1.5 | |
| Cetiol^{®} SB 45 | | | 1.5 | | | | 2 | | | | |
| Cegesoft^{®} C 17 | | | | | | | | | | | 2 |
| Myritol^{®} PC | | | | | 5 | | | | | | |
| Myritol^{®} 331 | 2 | 5 | 1 | | | 6 | | 6 | | | |
| Finsolv^{®} TN | | | 2 | | | 2 | | | | | |
| **Ester mixture of example 1** | 4 | 3 | 4 | 5 | 4 | 4 | 4 | 6 | 8 | 3 | 5 |
| Cetiol^{®} Sensoft | 2,0 | | | | | 2,0 | | | | 3,0 | |
| Cetiol^{®} CC | | 3 | | | | | 4 | | | | 5 |
| Cetiol^{®} OE | | | 2,0 | | | | | | 4 | | |
| Dow Corning DC^{®} 245 | | | 2 | | 1 | 1 | | | | | |
| Dow Corning DC^{®} 2502 | | | | | 2 | 1 | | | | | |
| Prisorine^{®} 3758 | | | | | | 1 | | | | | |
| Silicone Oil Wacker AK^{®} 350 | 0.5 | 0.5 | 0.5 | | | 1 | | | | | |
| Cetiol^{®} 868 | | | | | 2 | | 4 | | | | |
| Cetiol^{®} J600 | 2 | | 3 | | 3 | 2 | | | | 5 | |
| Ceraphyl^{®} 45 | | | | | | | 3 | | | | |
| Mineral Oil | | | | 9 | | | | | | | |
| Cetiol^{®} SN | | | 5 | | | | | | | | |
| Cetiol^{®} B | | | | | | | | 4 | | 2 | |
| Eutanol^{®} G | | 2 | | 3 | | | | | | | |
| Cetiol^{®} PGL | | | | | | | | | 5 | 5 | |
| Dry Flo^{®} Plus | 5 | | | | | | 1 | | | | |
| SFE 839 | 5 | | | | | | | | | | 2 |
| Almond Oil | | | | | | | 1 | | | | |
| Insect Repellent^{®} 3535 | | 2 | 4 | | | 2 | | | | 3 | |
| N,N-Diethyl-m-toluamide | | 2 | | | | | | | | 3 | |
| Photonyl^{®} LS | 2 | 2 | | | | 2 | | | | | |
| Panthenol | 1 | | | | | | | | | | |
| Bisabolol | 0.2 | | | | | | | | | | |
| Tocopherol / Tocopheryl Acetate | 1 | | | | | | | | | | |
| Veegum^{®} Ultra | | | | | | | | | 1 | | |
| Keltrol^{®} T | | | 0.4 | | | | | | 0.5 | | |
| Cosmedia^{®} SP | | 0.3 | | 0.2 | 0,2 | | | | 0.2 | 0.3 | |
| Pemulen^{®} TR 2 | 0.3 | | | | | | | 0.3 | | | |
| Carbopol^{®} Ultrez 10 | | | | | | 0.2 | | | | | |
| Rheocare® C Plus | | | 0.3 | | 0.2 | | | | | | |
| Ultragel™ 300 | | | | | | | | | 0,2 | | |
| Ethanol | | | | | | | | | | 10 | |
| Butylene glycol | | | | 4 | 3 | | 2 | 5 | 2 | | |
| Glycerin | 2 | 5 | 5 | | 3 | 3 | 2 | | 4 | | 3 |
| Water, Preservatives, NaOH | ad 100, q.s., pH 6,5-7,5 | | | | | | | | | | |

**Table 2: O/W Body Care Emulsions**

| **Ingredients** | **12** | **13** | **14** | **15** | **16** | **17** | **18** | **19** | **20** | **21** | **22** |
|---|---|---|---|---|---|---|---|---|---|---|---|
| **C - Cream, L - Lotion** | **C** | **C** | **L** | **C** | **L** | **C** | **C** | **L** | **L** | **L** | **C** |
| Eumulgin^{®} VL 75 | | | 1 | | | | | | | | 1 |
| Generol^{®} R | | | | | | 0.3 | | | | | |
| Eumulgin^{®} B2 | | | | | | | | | | 2 | |
| Tween^{®} 60 | | | | 2 | | | | | | 1 | |
| Cutina^{®} E 24 | | | | 0.5 | | | | | | 1 | |
| Lanette^{®} E | 0.5 | | | | | | | | | | |
| Amphisol^{®} K | | 0.5 | | | | | | | 0.1 | | |
| Sodium Stearate | | | | | 1 | | | | | | |
| Emulgade^{®} PL 68/50 | | 3 | | | | 3,0 | | 1 | 2 | | |
| Eumulgin^{®} SG | | | | | | | 0,5 | | | | 0,5 |
| Eumulgin^{®} Prisma | | | 0,5 | | | 0,2 | | 0,2 | | | |
| Inwitor 372 P | 3 | 2 | 3 | | 3 | | 1 | 1 | | | |
| Tego^{®} Care 450 | | | | | 1 | 2,0 | 3.8 | 1 | 1 | | |
| Cutina^{®} PES | 2 | | 1 | | 2.5 | 2 | | 1.2 | | 1.5 | 3 |
| Cutina^{®} MD | 3 | 1 | | 4 | | | | | | | |
| Lanette^{®} 14 | | 2 | | | 1 | | | 2 | | 1 | |
| Lanette^{®} | 2 | | | 2 | | 3 | 1 | | 1 | 1 | 6 |
| Novata^{®} AB | | | | | | | | | 1 | 1 | |
| Emery^{®} 1780 | | | | | | | | | | | 0.5 |
| Lanolin, water-free, USP | | | | | | 4 | | | | | |
| Cosmedia^{®} DC | | | 1 | | | 1.5 | | | 1 | 1 | |
| Cetiol^{®} SB 45 | | | | | | | 2 | | | | |
| Cegesoft^{®} C 17 | 4 | | | | | | | | | | |
| Myritol^{®} PC | 6 | | | | | 5 | | | 5 | | |
| Myritol^{®} 331 | | | 5 | | | | 7 | | | 10 | 3 |
| Finsolv® TN | | 5 | | 4 | 5 | | | | | | 1 |
| **Ester mixture of example 1** | 5 | 2 | 4 | 6 | 2 | 5 | 4 | 3 | 3 | 8 | 2 |
| Cetiol^{®} Sensoft | | 2 | | 3 | | | | | | | |
| Cetiol^{®} CC | | | 4 | | | | | 3 | | | |
| Cetiol^{®} OE | 2.5 | | | | | | 2 | | 5 | | |
| Dow Corning DC^{®} 245 | | | | | 1 | 3 | | | | | 2 |
| Dow Corning DC^{®} 2502 | | 1 | | | 1 | | | | | | 3 |
| Prisorine^{®} 3758 | 3 | | | | | | | | | | 2 |
| Silicone Oil Wacker AK^{®} 350 | | | | | 1 | | | | | | 1 |
| Cetiol^{®} 868 | | 2 | | | | | | | | | |
| Cetiol^{®} J 600 | | 2 | | 2 | | | | | | | |
| Geraphyl^{®} 45 | | | | | | | 3 | | | | |
| Cetiol^{®} SN | | | | 5 | | | | | | | |
| Cetiol^{®} B | | | | | | 5 | | 4 | | | 3 |
| Eutanol^{®} G | | 3 | | | 5 | | | | | | |
| Cetiol^{®} PGL | | | | | | | | 5 | 2 | | |
| Dry Flo^{®} Plus | | 1 | | | | | | | | | 1 |
| SFE839 | 1 | 1 | | | | | | | | | |
| Almond Oil | | | | | | 2 | | | | | |
| Photonyl^{®} LS | | | | | | 2 | | | | | |
| Panthenol | 1 | | | | | | | | | | |
| Bisabolol | 0.2 | | | | | | | | | | |
| Tocopherol / Tocopheryl Acetate | 1 | | | | | | | | | | |
| Veegum^{®} Ultra | | | | | | | | | 1 | | |
| Keltrol^{®} T | | | | | | | | | 0.5 | | |
| Cosmedia^{®} SP | 0.1 | | 1 | | 0.2 | 0.2 | 0.2 | 0.2 | | | 0.5 |
| Carbopol^{®} ETD 2001 | | | | 0.3 | | | | | | | |
| Pemulen^{®} TR 2 | | | | | | 0.3 | | | | | |
| Rheocare^{®} C Plus | 0,2 | 0.3 | | | | | | | | | |
| Ultragel™ 300 | | | | 0,4 | | 0,3 | | | | 0,4 | |
| Ethanol | | 5 | | 8 | | | | | | | 10 |
| Butylene glycol | 5 | | | 3 | 3 | | | | | 8 | |
| Glycerin | 2 | 4 | 3 | 3 | | 7 | 5 | 3 | 5 | | |
| Water, Preservatives, NaOH | ad 100, q.s., (pH 6,5-7,5) | | | | | | | | | | |

**Table 3: O/W Body Care Emulsions**

| **Ingredients** | **23** | **24** | **25** | **26** | **27** |
|---|---|---|---|---|---|
| **C - Cream, L - Lotion, SC = Sprayable Cream** | SC | C | C | L | C |
| Dehyquart^{®} C 4046 | 6 | | | 3 | |
| Cutina^{®} GMS-SE | | | | | 5.5 |
| Cutina^{®} FS 45 | | | | | 1.5 |
| Eumulgin^{®} B2 | | 1 | | | |
| Eumulgin^{®} SG | | | 0,2 | | |
| Eumulgin^{®} Prisma | | 0,2 | | | |
| Inwitor 372 P | | | 2 | | |
| Cutina^{®} PES | 3 | 2 | 2 | 2 | 2 |
| Cutina^{®} MD | | 1,5 | | | |
| Cosmedia^{®} DC | | | | 0.5 | |
| Cegesoft^{®} PS 6 | | | | 4.5 | |
| Cegesoft^{®} SH | | 7 | 3 | | |
| Myritol^{®} 331 | | | 5 | 4.5 | |
| **Ester mixture of example 1** | 4 | 5 | 4 | 3 | 4 |
| Cetiol^{®} Sensoft | | 2 | | | |
| Cetiol^{®} CC | | | 3 | | |
| Cetiol^{®} OE | | 1 | | | |
| Silicone Oil Wacker AK^{®} 350 | | | | | 0.5 |
| Paraffin Liquid | | | | | 2 |
| Isopropyl Palmitate | | | | 2 | |
| Cetiol^{®} 868 | | 2 | 4 | | |
| Cetiol^{®} SN | 4 | | | | 3 |
| Eutanol^{®} G | | | | | 3 |
| Almond Oil | | 7 | | | |
| Panthenol | 1 | 0.2 | 1 | | |
| Bisabolol | 1 | | | | |
| Tocopherol / Tocopheryl Acetate | 0.2 | | | | |
| Keltrol^{®} T | 1 | | | | |
| Ultragel™ 300 | 0,1 | | | 0.45 | |
| Cosmedia^{®} SP | | 1 | 0.7 | | |
| Glycerin | 2 | 5 | 5 | 5 | |
| Water, Preservatives, NaOH | ad 100, q.s. | | | | |

**Table 4: W/O Body Care Emulsions**

| Ingredients (INCl) | **1** | **2** | **3** | **4** | **5** | **6** | **7** | **8** | **9** | **10** | **11** |
|---|---|---|---|---|---|---|---|---|---|---|---|
| L = Lotion, C = Cream | C | L | C | L | C | L | L | L | C | C | C |
| Dehymuls^{®} PGPH | 1 | 2 | 1 | 2 | 3 | 1 | 1 | 2 | | | 1 |
| Monomuls^{®} 90-O18 | 2 | | | | | | | | 2 | | 2 |
| Lameform^{®} TGI | 4 | 1 | | | 3 | | | 1 | 4 | 3 | |
| Abil^{®} EM 90 | | | | | | | 4 | | 1 | | |
| Isolan GPS | | | 2 | | 2 | | | | | 1 | |
| Isolan^{®} PDI | | | | | | 4 | | | | | 1 |
| Glucate^{®} DO | | | | | | | | | | | |
| Arfacel^{®} 83 | | | 4 | | | | | | | | |
| Dehymuls^{®} LE | | 1 | 1 | 2 | | | | | | 1 | 1 |
| Dehymuls^{®} HRE 7 | | | | | | | | 4 | | 1 | |
| Zinc Stearate | 2 | 1 | | 1 | 1 | | | 1 | 1 | 1 | |
| Mlcrocristalline Wax | | | 5 | | | 2 | | | | | 5 |
| Bees wax | 4 | | | 1 | | | | 1 | 4 | 7 | |
| Tego Care^{®} CG | | | | | 1 | | | | | | 0,5 |
| Prisorine^{®} 3505 | | | 1 | 1 | | 1 | 1 | | | | 1 |
| SFE^{®} 839 | | | | | | | 3 | | | | |
| Emery^{®} 1780 | 1 | | | | | | | | | | 1 |
| Anhydrous Lanolin USP | | | 5 | | | | | | | 4 | |
| **Ester mixture of example 1** | 3 | 4 | 2 | 6 | 6 | 2 | 2 | 6 | 3 | 8 | 1 |
| Cegesoft^{®} C 17 | | | 3 | | | | | | | 1 | |
| Myritol^{®} PC | | | | | | 2 | | 4 | | | |
| Myritol^{®} 331 | 6 | | | | 2 | 6 | 2 | | | | 8 |
| Finsolv^{®} TN | | | | 5 | | 2 | 5 | | | | |
| Cetiol^{®} A | | 6 | | | | 4 | | | | | |
| Cetiol^{®} Sensoft | | | | 6 | 4 | | | | | 4 | |
| Cetiol^{®} CC | | 8 | | | 2 | 2 | 2 | | | | 5 |
| Cetiol^{®} SN | | 5 | | | | | | 3 | | | |
| Cetiol^{®} OE | 3 | | | | 4 | | 2 | | 4 | 2 | |
| Dow Corning DC^{®} 244 | | | | | 1 | | 2 | | | | |
| Dow Corning DC^{®} 2502 | | | 1 | | 2 | | | | | | |
| Prisorine^{®} 3758 | | | | | 3 | | | | | | |
| Silicon Oil Wacker AK^{®} 350 | | | | 4 | | | | 3 | | | |
| Cetiol^{®} 868 | | | | | | | | | | 2 | 7 |
| Cetiol^{®} J 600 | | | 4 | | | 2 | | | | | |
| Ceraphyl^{®} 45 | | | | 2 | | | | 2 | | 6 | |
| Mineral oil | | | | | 4 | | | | | | |
| Cetiol^{®} B | | | 2 | 4 | | | | | | 3 | |
| Eutanol^{®} G 16 | | 1 | | | | | | | | 3 | |
| Eutanol^{®} G | | | 3 | | | | | 8 | | | |
| Cetiol^{®} PGL | | | | | | 4 | | | 9 | | |
| Almond Oil | | | | | 1 | | 5 | | | | |
| Insect Repellent^{®} 3535 | 2 | | | | | | | | | | |
| Unirep^{®} U-18 | | | | 3 | | | | 5 | | | |
| Photonyl^{®} LS | 2 | 2 | | | | | | | | | |
| Panthenol | 1,0 | | | | | | | | | | |
| Bisabolol | 0,2 | | | | | | | | | | |
| Copherol^{®} 1250 C | 1 | | | | | | | | | | |
| MgSO₄ x 7 H₂O | 1 | | | | | | | | | | |
| Bentone^{®} 38 | | | | | 1 | | | | | | |
| Propylene Carbonate | | | | | 0,5 | | | | | | |
| Ethanol | | | | | | | | | | 8 | |
| Butylene Glycol | | | 2 | 6 | | | 2 | 5 | | | 2 |
| Glycerin | 5 | 3 | 3 | | 5 | 3 | 2 | | 10 | 4 | |
| Water, Preservative | ad 100, q.s. | | | | | | | | | | |

**Table 5: O/W Sun Care Emulsions**

| **Ingredients** | **1** | **2** | **3** | **4** | **5** | **6** | **7** | **8** | **9** | **10** | **11** |
|---|---|---|---|---|---|---|---|---|---|---|---|
| **C - Cream, L - Lotion** | **L** | **C** | **S** | **L** | **C** | **L** | **L** | **C** | **L** | **C** | **L** |
| Eumulgin^{®} VL 75 | 2,0 | | | | | | | 2 | | | 2 |
| Eumulgin^{®} B2 | | | | 0.5 | | | | | | | |
| Tween^{®} 60 | | | | 0.2 | | | | | | | |
| Myrj^{®} 51 | | | | 0.5 | | | | | | | |
| Cutina^{®} E 24 | | | | 0.1 | | | | | | | |
| Hostaphat^{®} KL 340 N | | | | | | | | | 1.6 | | |
| Lanette^{®} E | | | 0.3 | | | | | | | | |
| Amphisol^{®} K | | | | | | | | | | 1 | |
| Sodium Stearate | | | | | | | 1 | | | | |
| Emulgade^{®} PL 68/50 | | 2 | 1 | | | 2 | 2 | | | 2 | |
| Imwitor 372 P | | 2 | | | | 1 | | 2 | | | |
| Eumulgin^{®} SG | | 0,5 | | | | 0.1 | | 0,2 | | | |
| Eumulgin^{®} Prisma | 0,1 | | | | 0,75 | | | | | | |
| Tego^{®} Care 450 | | | | | | 2 | | | | 1 | 2.5 |
| Cutina^{®} PES | 2 | | 2.5 | 1 | 2.5 | | 2.5 | | 2.5 | 1.7 | 1.5 |
| Cutina^{®} MD | 2 | | 1 | 2 | | | 2 | | | 6 | |
| Lanette^{®} 14 | 1 | | | 1 | | | | 2 | | | 2 |
| Lanette^{®}O | 1 | 6 | | | 5 | 2 | | 2 | | | |
| Cosmedia^{®} DC | 1 | 1.5 | | 1 | 1 | | 2 | 2 | | | 2 |
| Antaron^{®}V 216 | | | 2 | | | 1.5 | | | 1 | 1 | |
| Emery 1780 | | | | | 0.5 | 0.5 | | | | | |
| Lanolin, water-free USP | | | | | | | 5 | | | | |
| Myritol^{®} PC | | | | | 5 | | | | | | |
| Myritol^{®} 331 | | | 8 | | | 6 | | 10 | | 2 | |
| Finsolv^{®} TN | | | 1 | | | | | 1 | | | |
| **Ester mixture of example 1** | 5 | 2 | 3 | 5 | 3 | 4 | 3 | 2 | 5 | 2 | 5 |
| Cetiol^{®} Sensoft | | 2,5 | | | 2 | | | | 3 | | |
| Cetiol^{®} CC | | | 2 | | | | 1 | | | | |
| Cetiol^{®} OE | | | 3 | | | | | | 2 | 3 | |
| Dow Corning DC^{®} 244 | 4 | | 1 | | | | | 2 | | | 2 |
| Dow Corning DC^{®} 2502 | | 1 | | | 2 | | | | | | |
| Squatol^{®} S | | | | | | | 4 | | | | |
| Silicone Oil Wacker AK^{®} 350 | | 2 | | | | | | | | | |
| Cetiol^{®} 868 | | | | | 2 | | 4 | | | | 2 |
| Cetiol^{®} J 600 | | | | | 3 | 2 | | | | 5 | |
| Mineral Oil | | | | 4 | | | | | | | |
| Cetiol^{®} B | | | 1 | | | | | | | 2 | |
| Eutanol^{®} G | | | | 2 | | | | | 4 | | |
| Eutanol^{®} G 16 | 4 | | | | | 4 | | | | | |
| Cetiol^{®} PGL | | 5 | | | | | | | | 5 | |
| Almond Oil | | | 2 | | | | 1 | | | | |
| Photonyl^{®} LS | | | | 2 | | | | | | 2 | |
| Panthenol | 1 | | | | | | | | | | |
| Bisabolol | 0.2 | | | | | | | | | | |
| Tocopherol / Tocopheryl Acetate | 1 | | | | | | | | | | |
| Photonyl^{®} LS | | | | | | | | | | | |
| Neo Hefiopan^{®} AP (Na-salt) | | 1 | | | | | | | 1 | | |
| Neo Heliopan^{®} Hydro (Na-salt) | 2 | | 2.2 | | | | | | 1 | | |
| Neo Heliopan^{®} 303 | 3 | 5 | 9 | 4 | | | | | | | |
| Neo Heliopan^{®} BB | | | | | 1 | | | | | | 2 |
| Neo Heliopan^{®} MBC | 2 | | | 3 | | 2 | 2 | 2 | | | 1 |
| Neo Heliopan^{®} OS | | | | | | | | | 10 | 7 | |
| Neo Heliopan^{®} E 1000 | | 7.5 | | 6 | | | | | | | 6 |
| Neo Heliopan^{®} AV | | | 7.5 | | | 7.5 | 4 | 5 | | | |
| Uvinul^{®} A Plus | | | | 2 | 1 | | | | | | |
| Uvinul^{®} T 150 | 2 | | | | 2.5 | | | 1 | | | |
| Tinosorb^{®} M | | | 3 | | | | 2 | | | | 3 |
| Tinosorb^{®} S | | | 1 | | | | 1,5 | | | | |
| Uvasorb^{®} HEB | | 1 | | | 1 | | | | | | |
| Parsol^{®} 1789 | | 1 | 1 | | | | 2 | | 2 | 2 | |
| Zinkoxid NDM | 10 | | 5 | | | 10 | | 3 | | 5 | 4 |
| Eusolex^{®} T 2000 | | | | | 5 | | 3 | 3 | | | 4 |
| Veegum^{®} Ultra | 1.5 | | 0.75 | | | | | 1 | 1 | | |
| Keltrol^{®} T | 0.5 | | 0.25 | | | | | 0.5 | 0.5 | | |
| Cosmedia^{®} SP | 0,1 | 0.5 | | | 0.5 | | 0.2 | 0.2 | | 0.2 | 0.2 |
| Ultragel™ 300 | | | | 0.2 | | 0.2 | | | 0.1 | | |
| Rheocare^{®} C plus | | | | | | | | | | 0.3 | 0.2 |
| Ethanol | | | | | | | | | | 10 | |
| Butylene glycol | | 2 | | 4 | 3 | | 2 | 5 | 2 | | 2 |
| Glycerin | 5 | 5 | 5 | | 3 | 3 | 2 | | 4 | | 3 |
| Preservatives, NaOH, Water | q.s,ad 100 | | | | | | | | | | |

**Table 6: O/W Sun Care Emulsions**

| **Ingredients** | **12** | **13** | **14** | **15** | **16** | **17** | **18** | **19** | **20** | **21** | **22** |
|---|---|---|---|---|---|---|---|---|---|---|---|
| **C - Cream, L - Lotion** | **L** | **C** | **L** | **C** | **L** | **C** | **S** | **C** | **C** | **L** | **L** |
| Eumulgin^{®} VL 75 | | | 4 | | 1.8 | | | | | | |
| Eumulgin^{®} B2 | | | | | | | | | | 0.2 | |
| Tween^{®} 60 | | | | | | | | | | 0.3 | |
| Cutina^{®} E 24 | | | | | | | | | | 0.5 | |
| Hostaphat^{®} KL 340 N | | | | | | | | | | | 0.5 |
| Imwitor 372 P | 2 | | | 2 | | | 2 | | 2.0 | | |
| Eumulgin^{®} SG | | | | 0,1 | | 0,2 | | | | | |
| Eumulgin^{®} Prisma | | 0,3 | 0,2 | | | | | | | | |
| Lanette^{®} E | | | | | | | 0.1 | | 0.5 | | |
| Amphisol^{®} K | 0.5 | | | | | | | 1 | | | |
| Sodium Stearate | | | | | 1 | | | | | | |
| Emulgade^{®} PL 68/50 | | 1.5 | | 2 | | 3 | | 2 | | | |
| Tego^{®} Care 450 | 1 | | | | | 2 | | 2 | 0.8 | | |
| Cutina^{®} PES | 2 | 2 | 2.5 | 1.5 | 2 | 2 | 2.5 | 3 | | 1.5 | 1.5 |
| Cutina^{®} MD | 1 | | | 4 | 1 | 3 | | | 5 | | 1 |
| Lanette^{®} 14 | | 2 | | | | | | | | 1 | |
| Lanette^{®} O | | 2 | | 2 | | | | 2 | 1 | 1 | |
| Allianz^{®} OPT | 1 | | | 1 | 1 | | | 2 | | | 2 |
| Cosmedia^{®} DC | | 1.5 | 2 | | | 1.5 | 2 | | 1.5 | 1.5 | |
| Emery^{®} 1780 | | | | 1 | 1 | | | | | | |
| Lanolin, water-free, USP | | | | | | 1 | 1 | | | | |
| Myritol^{®} PC | | | | | | | | | 3 | | |
| Myritol^{®} 331 | 12 | | 12 | | | 8 | 8 | | | 5 | 3 |
| Finsolv^{®} TN | | | | | 3 | | | | 3 | | |
| **Ester mixture of example 1** | 4 | 2 | 3 | 5 | 3 | 2 | 4 | 3 | 2 | 5 | 3 |
| Cetiol^{®} Sensoft | | | 3 | | | 5 | | | | | 2 |
| Cetiol^{®} CC | 2 | | | | | | 1 | | | | |
| Cetiol^{®} OE | | | | | 2 | | | | | | 2 |
| Dow Corning DC^{®} 244 | | | | | 1 | | | | | | |
| Dow Corning DC^{®} 2502 | | 1 | | | | | | 3 | | | |
| Ceraphyl^{®} 45 | | | | | | | | | | 2 | 2 |
| Silicone oil Wacker AK^{®} 350 | | | | | 1 | | | | | | |
| Cetiol^{®} 868 | | 2 | | | | | | | | | |
| Cetiol^{®} J 600 | | 2 | | | | | | | | | |
| Mineral Oil | | | | 5 | | | | | | | |
| Cetiol^{®} B | 4 | | 4 | | | | | 4 | | | |
| Eutanol^{®} G | | 3 | | | | 3 | | | | | |
| Eutanol^{®} G 16 S | 10 | | | | | | | | | | |
| Cetiol^{®} PGL | | | | | | | | | 2 | | |
| Photonyl^{®} LS | | | | | | | | | | 2 | |
| Panthenol | 1 | | | | | | | | | | |
| Bisabolol | 0,2 | | | | | | | | | | |
| Tocopherol/ Tocopheryl Acetate | 1 | | | | | | | | | | |
| Neo Heliopan^{®} Hydro (Na-salt) | | | | | | | | | | 3 | |
| Eusolex^{®} OCR | 6 | | 9 | | 5 | 7 | 9 | | 4 | | 7 |
| Neo Heliopan^{®} AP (Na-salt) | | | | 0.5 | | 1 | | | | | |
| Neo Heliopan^{®} BB | | | | | | | | 1 | 1 | | 1 |
| Neo Heliopan^{®} MBC | | 2 | | 1 | | | | 3 | 1 | | 3 |
| Neo Heliopan^{®} OS | 2 | | | | | | | | 7 | | |
| Neo Heliopan^{®} E1000 | | 4 | | | | | | 5 | | | |
| Neo Heliopan^{®} AV | | 4 | 7.5 | 5 | | | | 5 | 4 | 7.5 | |
| Uvinul^{®} A PLUS | | | | | 1 | | 2 | | | | |
| Uvinul^{®} T 150 | 1 | | | | | | | | 1.3 | 1 | 1 |
| Tinosorb^{®} M | | | 6,5 | | | | | | | 4 | |
| Tinosorb^{®} S | | | 1 | | 2 | | | | | | |
| Uvasorb^{®} HEB | 1 | | | | | | | | | | 2 |
| Parsol^{®} 1789 | 1 | | | | | | | | 2 | | 1 |
| Z-Cote^{®} HP 1 | 7 | 2 | 5 | | | 7 | 5 | | 6 | 2 | |
| Eusolex^{®} T 2000 | 5 | 2 | | | 10 | | | 10 | | 2 | |
| Veegum^{®} Ultra | 1.5 | | 1.5 | | | 1.5 | 1.2 | | 1 | | |
| Keltrol^{®} T | 0.5 | | 0.5 | | | 0.5 | 0.4 | | 0.5 | | |
| Cosmedia^{®} SP | | | 0.2 | | | | 0.1 | | | | |
| Pemulen^{®} TR 2 | | 0.3 | | 0.3 | | | | 0.2 | | | |
| Ultragel™ 300 | | | | | | | | | | 0.2 | 0.3 |
| Rheocare^{®} C Plus | | | | 0.3 | | | 0,1 | | | | |
| Ethanol | | 5 | | 8 | | | | | | | |
| Butylene glycol | 1 | | | 3 | 3 | | | | | 8 | 1 |
| Glycerin | 2 | 4 | 3 | 3 | | 3 | 3 | 3 | 5 | | 3 |
| Water / Preservatives / NaOH | ad 100/q.s./q.s | | | | | | | | | | |

**Table 7: W/O Sun Care Emulsionen**

| **Ingredients** | **23** | **24** | **25** | **26** | **27** | **28** | **29** | **30** | **31** | **32** | **34** |
|---|---|---|---|---|---|---|---|---|---|---|---|
| **C - Cream, L - Lotion** | **C** | **L** | **C** | **L** | **C** | **L** | **L** | **L** | **L** | **C** | **C** |
| Dehymuls^{®} PGPH | 4 | 3 | 1 | 3 | 2 | 1 | 1 | 1 | | | |
| Monomuls^{®} 90-018 | | 1 | 2 | | | | | | 2 | 4 | |
| Lameform^{®} TGI | 2 | | | | 3 | | | | | 1 | 3 |
| Abil^{®} EM 90 | 2 | | | | | | 4 | | 1 | | 1 |
| Isolan GPS | | | 4 | | 3 | | | 2 | | | |
| Isolan^{®} PDI | | | | | | 4 | | 2 | | | |
| Zinc Stearate | 1 | | | 1 | 1 | | | 1 | | 1 | |
| Beeswax | 1 | | 5 | 1 | 3 | | | 2 | | 7 | 5 |
| Tego^{®} Care CG | | | | | 1 | | | | | | 0.5 |
| Cutina^{®} PES | | | 2 | | | 1 | 1 | | | | |
| Prisorine^{®} 3505 | | | 1 | | | 1 | 1 | | | | 1 |
| Cosmedia^{®} DC | 3 | 4 | 2 | 1 | 1 | 2 | 2 | 2 | 3 | 1 | 1 |
| Myritol^{®} 331 | 2 | | | | 3 | 3 | | | | | 8 |
| Finsolv^{®} TN | | | | 2 | | | | | | | |
| **Ester mixture of example 1** | 5 | 4 | 2 | 3 | 4 | 3 | 5 | 5 | 4 | 4 | 5 |
| Cetiol^{®} Sensoft | | | | 3 | | | 5 | | | 3 | |
| Cetiol^{®} CC | 5 | | | | | 2 | | | 2 | 3 | |
| Tegosoft^{®} DEC | | 4 | | | 2 | | | | | | |
| Cetiol^{®} OE | | | | | 4 | | 5 | | | 2 | |
| Dow Corning^{®} DC 244 | | | 3 | | | | 2 | | 4 | | |
| Dow Corning^{®} DC 2502 | 1 | | 1 | | 2 | 1 | | | | | 1 |
| Silicone oil Wacker AK 350 | | 1 | | 4 | | | | 3 | | | |
| Cetiol^{®} PGL | | 3 | | | | 4 | | | 4 | | |
| Copherol^{®} F 1300 | 1 | | | | | | | | | | |
| MgSO₄*7H₂O | 1 | | | | | | | | | | |
| Neo Heliopan^{®} Hydro (Na-salt) | 2 | | 2.2 | | 3 | 3 | | | 1 | | 2 |
| Neo Heliopan^{®} 303 | | 5 | | | | | | | 4 | | 4 |
| Uvasorb^{®} HEB | 1 | | | 1 | 1 | | | | | | 2 |
| Neo Heliopan^{®} MBC | 2 | | | | | 2 | 2 | 2 | | | |
| Uvinul^{®} A Plus | | | | | 2 | | | | 3 | 3 | |
| Neo Heliopan^{®} AP (Na-saft) | | 2 | 2 | | 1 | | | | 1 | | 6 |
| Neo Heliopan^{®} AV | 3 | | 4 | 6 | 4 | 7.5 | 4 | 5 | | | 1 |
| Uvinul^{®} T 150 | 1 | 1 | | | 2.5 | | | 1 | | | |
| Parsol^{®} 1789 | 2 | 1 | | | | | 2 | | 2 | 2 | |
| Zinkoxid NDM | | | | | | 10 | | 3 | | | 4 |
| Tinosorb^{®} M | | 3 | | 3 | | | | 2 | | 2 | |
| Tinosorb^{®} S | | 3 | | 3 | | | | 2 | | 2 | |
| Eusolex^{®} T Aqua | | | 8 | | | | | 5 | | | |
| Eusalex^{®} T 2000 | | | | | 5 | | 3 | 3 | | | 4 |
| Ethanol | | | | | | | | | | 8 | |
| Glycerin | 5 | 3 | 3 | 3 | 5 | 3 | 2 | 3 | 10 | 4 | 3 |
| Water, Preservatives | ad 100, q.s. | | | | | | | | | | |

**Table 8: W/O Sun Care Emulsionen**

| **Ingredients** | **12** | **13** | **14** | **15** | **16** | **17** |
|---|---|---|---|---|---|---|
| Dehymuls^{®} PGPH | 1 | 1 | | | 1 | 1 |
| Dehymuls^{®} LE | 1 | 2 | 1 | 1 | 1 | 1 |
| Abil^{®} EM 90 | | | | 4 | | |
| Isolan GPS | 3 | | 1 | 1 | | |
| Isolan^{®} PDI | | | 4 | | 2 | |
| Zinc Stearate | | 1 | | | 1 | |
| Beeswax | | 1 | | | 5 | |
| Cutina^{®} PES | | 1 | | 1 | | |
| Prisorine^{®} 3505 | | | 1 | 1 | | |
| Cosmedia^{®} DC | 4 | 1 | 2 | 2 | 2 | 3 |
| Myritol^{®} 331 | | | 3 | | | |
| Finsolv^{®} TN | | 2 | | | | |
| **Ester mixture of example 1** | 4 | 3 | 3 | 5 | 5 | 4 |
| Cetiol^{®} CC | | | 2 | | | 2 |
| Cetiol^{®} Sensoft | | 2 | | 2 | | 4 |
| Tegosoft^{®} DEC | 4 | 3 | | 5 | | |
| Cetiol^{®} OE | 2 | | | 5 | | |
| Dow Corning^{®} DC 244 | | | | 2 | | 4 |
| Dow Corning^{®} DC 2502 | | | 1 | | | |
| Silicone oil Wacker AK 350 | 1 | 4 | | | 3 | |
| Cetiol^{®} PGL | 3 | | 4 | | | 4 |
| Copherol^{®} F 1300 | 1 | | | | | |
| MgSO₄*7H₂O | 1 | | | | | |
| Neo Heliopan^{®} Hydro (Na-salt) | | | 3 | | | 1 |
| Neo Heliopan^{®} 303 | 5 | | | | | 4 |
| Uvasorb^{®} HEB | | 1 | | | | |
| Neo Heliopan^{®} MBC | | | 2 | 2 | 2 | |
| Uvinul^{®} A Plus | | | | | | 3 |
| Neo Heliopan^{®} AP (Na-salt) | 2 | | | | | 1 |
| Neo Heliopan^{®} AV | | 6 | 7.5 | 4 | 5 | |
| Uvinul^{®} T 150 | 1 | | | | 1 | |
| Parsol^{®} 1789 | 1 | | | 2 | | 2 |
| Zinkoxid NDM | | | 10 | | 3 | |
| Tinosorb^{®} M | 3 | 3 | | | 2 | |
| Tinosorb^{®} S | 3 | 3 | | | 2 | |
| Eusolex^{®} T Aqua | | | | | 5 | |
| Eusolex^{®} T 2000 | | | | 3 | 3 | |
| Glycerin | 3 | 3 | 3 | 2 | 3 | 10 |
| Water, Preservatives | ad 100, q.s. | | | | | |

**Table 9: Decorative Cosmetics - O/W Foundations**

| **Ingredients** | **1** | **2** | **3** | **4** | **5** | **6** | **7** | **8** |
|---|---|---|---|---|---|---|---|---|
| Cutina^{®} GMS-SE | 5,5 | | | | | | | 3,0 |
| Emulgade^{®} PL 68/50 | | 5,0 | | | | 2,0 | | |
| Eumulgin^{®} VL 75 | | | | 3,0 | | | 5,0 | |
| Tego Care^{®} 450 | | | | | | 2,0 | 2,0 | |
| Codesta^{®} F-50 | | | | | 6,0 | | | |
| Amphisol^{®} K | | | | 2,0 | | | | |
| Lanette^{®} E | | 0,25 | | | | | | |
| Eumulgin^{®} SG | | | | | 1,0 | | 1 | |
| Eumulgin^{®} Prisma | | | | | | 1,0 | | 0,75 |
| Imwitor 372 P | | 2 | | | | | 1 | |
| Cutina^{®} FS 45 | 1,5 | | | | | | | |
| Eumulgin^{®} B 2 | | | 2,0 | | | | | |
| Cutina^{®} PES | 2,0 | 1,0 | 2,0 | 4,0 | 2,0 | 1,0 | 2,5 | 2,0 |
| Lanette^{®} O | | | 2,0 | | | | | 1,0 |
| Cutina^{®} MD | | 0,5 | 3,0 | 3,0 | | | | |
| Cetiol^{®} LC | 4,0 | | | | | | | |
| Cosmedia^{®} DC | 0,5 | | | 1,0 | | | | 1,0 |
| **Ester mixture of example 1** | 4,0 | 5,0 | 4,0 | 2,0 | 7,0 | 5,0 | 10,0 | 4,0 |
| Cetiol^{®} Sensoft | 2,0 | | | | 3,0 | | | 2,0 |
| Tegosoft^{®} DEC | | 5,0 | | 2,0 | | 2,0 | | 2,0 |
| Cetiol^{®} CC | | | 2,0 | | 2,0 | | | |
| Dow Corning^{®} 245 | | 2,0 | | 2,0 | | | | |
| Eutanol^{®} G 16 | 4,0 | | | | | 3,0 | | |
| Myritol^{®} 331 | | 5,0 | | | 2,0 | 2,0 | 5,0 | |
| Uvinul^{®} T 150 | | | | 0,5 | | | | 0,5 |
| Uvasorb^{®} HEB | 2,0 | | | | | | 1,0 | 1,0 |
| Tinosorb^{®} M | | | 2,0 | | | | | 2,0 |
| Tinosorb^{®} S | | | | 3,0 | | | | 2,0 |
| Neo Heliopan^{®} AV | | | | 2,0 | | | 2,0 | |
| Heo Heliopan^{®} AP | | | | 1,0 | | | 1,0 | |
| Uvinul^{®} A Plus | | | 1 | | | | 2,0 | 2,0 |
| Microna^{®} Matte White | 5,0 | 5,0 | | 5,0 | 5,0 | 5,0 | | 5,0 |
| Microna^{®} Matte Black | 0,3 | 0,3 | 0,1 | 0,3 | 0,3 | 0,3 | 0,4 | 0,3 |
| Microna^{®} Matte Yellow | 3,0 | 3,0 | | 3,0 | 3,0 | 3,0 | 2,0 | 3,0 |
| Microna^{®} Matte Red | 0,6 | 0,6 | 1,0 | 0,6 | 0,6 | 0,6 | 1,0 | 0,6 |
| Ronasphere^{®} | 1,0 | 1,0 | | 1,0 | 1,0 | 1,0 | | 1,0 |
| Pigment White 6 | | | 6,0 | | | | 6,0 | |
| Dry Flow PC | | | | | | | 2,0 | 2,0 |
| Glycerin | 5,0 | 5,0 | 3,0 | 5,0 | 5,0 | 5,0 | 3,0 | |
| Cosmedia^{®} SP | | | 0,3 | | 0,2 | | | |
| Water, de-ionized, Preservative | ad 100 | | | | | | | |

**Table 10: Decorative Cosmetics - W/O Foundations**

| **Ingredients** | **1** | **2** | **3** | **4** | **5** | **6** | **7** | **8** |
|---|---|---|---|---|---|---|---|---|
| Dehymuls^{®} PGPH | 5,5 | | 4,0 | | | | | 3,0 |
| Lameform^{®} TGI | | 5,0 | | | | 2,0 | | |
| Abil^{®} EM 90 | | | | 3,0 | | | 5,0 | |
| Isolan^{®} GI 34 | | | | | | 2,0 | 2,0 | |
| Isolan^{®} PDI | | | | 1,0 | 6,0 | | | |
| Isolan^{®} GPS | 1,0 | 2,0 | | 1,0 | | | | |
| Admul^{®} WOL 1403 | | | | 2,0 | | | | |
| Dehymuls^{®} HRE 7 | | 1,0 | | | 1,0 | 1,0 | | |
| Monomuls^{®} 90-O18 | 1,5 | | | | | | | 2,0 |
| Cutina^{®} PES | 2,0 | 1,0 | 2,0 | 4,0 | 2,0 | 1,0 | 2,5 | 2,0 |
| Cera Bellina | | | 2,0 | | | | | 2,0 |
| Beeswax | | | 2,0 | | | 2,0 | | 1,0 |
| Microcrystalline Wax | | 1,5 | 3,0 | 3,0 | | | | |
| Cetiol^{®} LC | 4,0 | 5,0 | | | | | | |
| Cosmedia^{®} DC | 1,0 | | | | 0,5 | | 1,0 | |
| **Ester mixture of example 1** | 4,0 | 2,0 | 2,0 | 4,0 | 5,0 | 5,0 | 5,0 | 4,0 |
| Cetiol^{®} Sensoft | | 2,0 | | | 2,0 | | 5,0 | |
| Tegosoft^{®} DEC | | 3,0 | | | | 2,0 | | |
| Cetiol^{®} CC | | | | | 2,0 | | | 2,0 |
| Dow Corning^{®} 245 | | 2,0 | | 2,0 | | | | 2,0 |
| Eutanol^{®} G 16 | 4,0 | | | | 3,0 | 3,0 | | |
| Myritol^{®} 331 | | 5,0 | | | 2,0 | 2,0 | 5,0 | |
| Uvinul^{®} T 150 | | | | 0,5 | | | | 0,5 |
| Uvasorb^{®} HEB | | | 2,0 | | | | 1,0 | 1,0 |
| Tinosorb^{®} M | | | 2,0 | | | | | 2,0 |
| Tinosorb S | | | | 3,0 | | | | 2,0 |
| Neo Heliopan^{®} AV | | | | 2,0 | | | 2,0 | |
| Heo Heliopan^{®} AP | | | | 1,0 | | | 1,0 | |
| Uvinul^{®} A plus | | | 1,0 | | | | 2,0 | 2,0 |
| Microna^{®} Matte White | 5,0 | 5,0 | | 5,0 | 5,0 | 5,0 | | 5,0 |
| Microna^{®} Matte Black | 0,3 | 0,3 | 0,1 | 0,3 | 0,3 | 0,3 | 0,4 | 0,3 |
| Microna^{®} Matte Yellow | 3,0 | 3,0 | 3,5 | 3,0 | 3,0 | 3,0 | 2,0 | 3,0 |
| Microna^{®} Matte Red | 0,6 | 0,6 | 1.0 | 0,6 | 0,6 | 0,6 | 1,0 | 0,6 |
| Ronasphere^{®} | 1,0 | 1,0 | | 1,0 | 1,0 | 1,0 | | 1,0 |
| Pigment White 6 | | | 6,0 | | | | 6,0 | |
| Dry Flow PC | | | | | | | 2,0 | 2,0 |
| Glycerin | 5,0 | 5,0 | 3,0 | 5,0 | 5.0 | 5,0 | 3,0 | |
| Water, de-ionized, Preservative | ad 100 | | | | | | | |

**Table 11: Decorative Cosmetics- Lipsticks**

| **Ingredients** | **1** | **2** | **3** | **4** |
|---|---|---|---|---|
| Cutina^{®} LM conc | | | 10,0 | 36,0 |
| Candelilla Wax | 9,39 | 5,0 | 10,0 | |
| Carnauba wax | 2,85 | 7.0 | 5,0 | |
| Beeswax | 1,86 | 5,0 | 4,0 | |
| Cutina^{®} PES | 3,2 | 5,0 | 6,4 | 4,5 |
| Cetiol^{®} MM | | | 5,0 | |
| Cosmedia^{®} DC | 5,0 | 4,0 | 2,0 | 6,0 |
| **Ester mixture of example 1** | 7,0 | 6,0 | 3,0 | 5,0 |
| Cetiol^{®} Sensoft | 2,0 | | 4,5 | |
| Tegosoft^{®} DEC | 3,0 | 3,0 | 3,0 | 5,0 |
| Eutanol^{®}G | 10,97 | 12,0 | 12,0 | |
| Fitoderm^{®} | | | 4,0 | |
| Monomuls^{®} 90L 12 | | 3,0 | | |
| Dehymuls^{®} PGPH | | 4,0 | | |
| Castor Oil | 11,0 | 15,5 | 14,5 | 30,0 |
| Copherol^{®} F 1300 | 1,0 | 1,0 | 1,0 | 1,0 |
| Cosmetic white C47056 | 5,0 | 2,0 | 5,0 | |
| FDC Yellow 6 Al Lake C705270 | 7,0 | 7,0 | 8,0 | |
| DC Red 7 Ca Lake C 19003 | 6,0 | 4,5 | 1,1 | 2,9 |
| Irodin 100 Silverpearl | | | | 9,6 |
| Hydagen^{®} CMF | | 10,0 | | |
| Irwinol^{®} LS 9319 | 1,0 | | 3,0 | |
| Mineral Oil | 12,8 | | | |
| Petrolatum | 6,84 | 3,0 | | |
| Ceresin | 2,75 | | | |
| Microcrystalline Wax | 2,45 | | | |
| Colophane Claire type Y | 1,89 | | | |

**Tabelle 12: AP/Deo Concepts**

| **Ingredients (INCI)** | **1** | **2** | **3** | **4** | **5** | **6** | **7** |
|---|---|---|---|---|---|---|---|
| Glyceryl Stearate, Ceteareth-20, Ceteareth-12, Cetearyl Alcohol, Cetyl Palmitate (Emulgade^{®} SE) | 6 | | | 4,5 | | 6 | |
| Ceteareth-20 (Eumulgin^{®}B2) | | | | 1 | | | |
| Glyceryl Stearate Citrate (Imwitor 372 P) | | 4,0 | | | | | |
| Polyglyceryl-3 Diisostearate (Lameform^{®} TGI) | | | 3 | | | | |
| Cocoglycerides (Novata^{®} AB) | | | | | | | 4 |
| Stearyl alcohol (Lanette^{®} 18) | | | | | 10 | | |
| Hydrogenated Castor Oil (Cutina^{®} HR) | | | | | 3,7 | | 6,5 |
| Polyglyceryl-2 Dipolyhydroxystearate (Dehymuls^{®} PGPH) | | | 1 | | | | |
| Sodium Stearoyl Glutamate (Eumulgin^{®}SG) | | 0,2 | | | | | |
| Disodium Cetearyl Sulfosuccinate (Eumulgin^{®}Prisma) | 0,3 | | | | | | |
| Sodium Cetearyl Sulfate (Lanette^{®} E) | | | | | | 0,3 | |
| Pentaerythrityl Distearate (Cutina^{®} PES) | 5 | 1 | 2 | 1 | 4,7 | 5 | 4 |
| Behenyl Alcohol (Lanette^{®} 22) | 2 | 1 | | | | 4 | |
| **Ester mixture of example 1** | 4 | 4 | 5 | 3 | 4 | 3 | 5 |
| Propylheptyl Caprylate (Cetiol^{®} Sensoft) | | 2 | | | 20 | | 10 |
| Dicaprylyl Carbonate (Cetiol^{®} CC) | | | 2 | | | | |
| Dicaprylyl Ether (Cetiol^{®}OE) | 2 | | | 2 | 5 | 3 | 4 |
| Cocoglycerides (Myritol^{®} 331) | | | | | | | |
| Diethylhexylcyclohexane (Cetiol^{®} S) | | | | 5 | 14,7 | | 25 |
| Cyclopentasiloxane | 3 | | 5 | | 14 | 3 | 14 |
| Cyclopentasiloxane and Dimethicone / Vinyldimethicone | | | 3 | | | | |
| Crosspolymer SFE 839 (GE Bayer) | | | | | | | |
| Dimethicone AK 350 | 1 | 2 | | | | | |
| Hydrogenated Dimer Dilinoleyl/Dimethylcarbonate | | | 1 | | 1 | 2 | 1 |
| Copolymer (Cosmedia^{®} DC) | 0,5 | | | 1,5 | | | |
| Triethyl Citrate (Hydagen^{®} C.A.T) | | | | 2 | | | |
| Tocopheryl Acetate | | | | | 1 | | |
| Aluminium Zirconium Tetrachlorohydrex GLY (Rezal 36) | 30 | | 40 | | 22,9 | 30 | 25 |
| Aluminum Chlorhydrate (Locron L) | | 20 | | 10 | | | |
| Chitosan (Hydagen^{®} DCMF) | 0,05 | | | | | | |
| Glycolic Acid | 0,02 | | | | | | |
| Glycerin | | | 5 | 5 | | | |
| Propylene Carbonate (Fluka) | | | | | | | 0,5 |
| Quatemium-18 Hectorite (Bentone 18) | | | | | | | 1 |
| Polyquaternium- 37 (Ultragel 37) | | 5 | | | | | |
| Talcum (Merck) | | | | | | 5 | 5 |
| MgSO₄x7H₂O | | | 1 | | | | |
| Water, Perfume, Preservatives | q.s. | q.s. | q.s. | q.s. | q.s. | q.s. | q.s. |

| | | | | | | | |
|---|---|---|---|---|---|---|---|
| 1/2- Antiperspirant / deo cream, 3 - Antiperspirant cream (W/0), 4- Antiperspirant / deo spray, 5 - Antiperspirant stick with vitamin E, 6 Antiperspirant cream, 7 - Antiperspirant cream 'Soft Solid' | | | | | | | |

**Table 13: Hair Care Conditioners**

| | **1** | **2** | **3** | **5** | **6** | **7** | **8** |
|---|---|---|---|---|---|---|---|
| Structure^{®}XL (*) Hydroxypropyl Starch Phosphate | 5.0 | 5.0 | 5.0 | 4.0 | | | |
| Emulgade^{®} Sucro Sucrose Polystearate, Hydrogenated Polyisobutene | | | | | 1.0 | 1.0 | 1.0 |
| Dehyquart^{®} L 80 Dicocoylethyl Hydroxyethylmonium Methosulfate, Propylene Glycol | 2.6 | 1.3 | 2.0 | | | 0.5 | 0.5 |
| Dehyquart^{®} F 75 Distearoylethyl Hydroxyethylmonium Methosulfate, Cetearyl Alcohol | | | 2.0 | | | | |
| Dehyquart^{®} C 304 Aqua, Cocamidopropyltrimonium Methosulfate, Propylene Glycol | | | | 3.7 | | 4.0 | 4.0 |
| **Ester mixture of example 1** | 1.0 | 3.0 | 1.0 | 0.5 | 2.0 | 1.5 | 1.5 |
| Dehyquart^{®}A CA Cetrimonium Chloride | | | | | 4.0 | | |
| DC 200 (***) Dimethicone | | | | 0.5 | | | |
| Lanette^{®}O Cetearyl Alcohol | | | 1.0 | | 4.0 | | |
| Lamesoft^{®}TM Benz | 4.0 | | | 1.0 | | | |
| Glycol Distearate, Coco Glucoside, Glyceryl Oleate, Glyceryl Stearate | | | | | | | |
| Gluadin^{®}WLM Hydrolyzed Wheat Protein | 1.0 | 1.0 | | 1.0 | | | 0.3 |
| Glycerin | | | 0.5 | | | | |
| Gluadin^{®} Soy Hydrolyzed Wheat Protein | | 0.5 | | | | | |
| Cacao Butter (**) Theobroma Cacao (Cocoa) Seed Butter | | | 0.5 | | | | |
| Herbalia^{®} Balm Mint Melissa Officinalis, Maltodextrin, Silica | | 0.01 | | | | | 0.02 |
| Ultragel™ 300 Polyquartemium-37 | | | | | | 0,2 | 0,2 |
| Perfume, preservative | q.s. | q.s. | q.s. | q.s. | q.s. | q.s. | q.s. |
| Deionized water | Up to 100 | Up to 100 | Up to 100 | Up to 100 | Up to 100 | Up to 100 | Up to 100 |

| | | | | | | | |
|---|---|---|---|---|---|---|---|
| (*)National Starch, (**) Nederland, (***) Dow Coming; pH adjusted to 3.5 - 5.0 | | | | | | | |

**Table 14: Hair Care Conditioners**

| | **9** | **10** | **11** | **12** |
|---|---|---|---|---|
| Dehyquart^{®} L 80 Dicocoylethyl Hydroxyethylmonium Methosulfate, Propylene Glycol | 1.3 | 1.3 | | 1.0 |
| Dehyquart^{®} F 75 Distearoylethyl Hydroxyethylmonium Methosulfate, Cetearyl Alcohol | 1.3 | 1.3 | 1.3 | 1.5 |
| Lanette^{®}O (Getearyl Alcohol) | 5.0 | 5.0 | 4.0 | 4.5 |
| Ester mixutre of example 1 | 1.0 | 1.0 | 1.0 | 0.5 |
| Cetiol^{®}SB 45 Butyrospermum Parkii (Shea Butter) | 4.0 | 4.0 | 2.0 | 4.5 |
| Gluadin^{®}Almound (Hydrolyzed Sweet Almound Protein) | | | 0.1 | 0.5 |
| ASCO BTAC (Behentrimonium Chloride) | | | 1.3 | |
| DC 949 (****)Amodimethicone, Trideceth-12, Cetrimonium Chloride | | 1.0 | | |
| Cegesoft^{®} PFO (Passiflora Incamata Seed Oil) | | | 2.0 | |
| Aloveria^{®} (Aloe Barbadensis) | 0.1 | | | |
| Sphingoceryl^{®} Veg: Octyldodecanol, Hydrogenated Coco Glycerides, Helianthus Annuus (Sunflower) Seed Extract | 1.0 | | | |
| Copherol^{®} 1250 (Tocopheryl Acetate) | 0.2 | | | |
| Ultragel^{™} 300 (Polyquartemium-37) | | 0.1 | | 0,2 |
| Perfume, preservative | q.s. | q.s. | q.s. | q.s. |
| Deionized water | Up to 100 | Up to 100 | Up to 100 | Up to 100 |

| | | | | |
|---|---|---|---|---|
| (****) Dow Coming; pH adjusted to 3.5 - 5.0 | | | | |

**Table 15: Hair Care Conditioners**

| | **13** | **14** | **15** | **16** |
|---|---|---|---|---|
| **Ester mixture of example 1** | 10 | 10.6 | 43.6 | 30 |
| Myritol^{®} 318 (Caprylic Capric Triglyceride) | | | 43.6 | 20 |
| Cetiol^{®} ISL (Isostearyl Lactate) | | | | 40 |
| DC 1501 (*) (Cyclomethicone, Dimethiconol) | 69.5 | | | |
| Emery^{®} 3004 (Hydrogenated Polydecene) | | 67.8 | | |
| DC 345 (*) Cyclomethicone | 20 | | | |
| Versagel MC 750 (**) Isohexadecene, Ethylene/Propylene/Styrene Copolymer, Butylene/Ethylene/Styrene Copolymer | | 21.3 | | |
| DC 556 (*) Phenyl Trimethicone | 0.5 | | | |
| Wacker HDK H 20 (***): Phenyl Trimethicone | | | 12.5 | 10 |
| Ultragel™ 300 (Polyquartemium-37) | 0,2 | | | 0,2 |
| Perfume | q.s. | q.s. | q.s. | q.s. |

| | | | | |
|---|---|---|---|---|
| (*) Dow Coming, (**) Penreco, (***) Wacker | | | | |

**Table 16: Hair Care Conditioners**

| **Ingredients (INCI)** | **17** | **18** | **19** | **20** | **21** |
|---|---|---|---|---|---|
| Cetearyl Alcohol (Lanette^{®} O) | 5,0 | 4,5 | | | |
| Glyceryl Stearate (Cufina^{®} MD) | 4,0 | | | 14,5 | |
| Cetearyl Alcohol (Lanette^{®} O) | | | | 7,0 | |
| Hydrogenated Castor Oil (Cutina^{®} HR) | | | | 2,5 | |
| Cetyl Palmitate (Cutina^{®} CP) | | 0,3 | | 7,0 | |
| Paraffin Oil | | | | 23,5 | |
| Vaseline | | | | 32,5 | |
| Wacker Silicon Oil AK 350 | | | | 0,5 | |
| **Ester mixture of example 1** | 3,0 | 0,2 | 1,5 | 2,0 | 5,0 |
| Oleyl Erucate (Cetiol^{®} J 600) | 2,0 | | | | |
| PEG-7 Glyceryl Cocoate (Cetiol^{®} HE) | | | | | 20,0 |
| Dimethicone (Dow Corning 200) | | 0,2 | | | |
| Ceteareth-12 (Eumulgin^{®} B1) | 1,0 | | | | |
| Ceteareth-20 (Eumulgin^{®} B2) | | 0,4 | | | |
| Ceteareth-30 (Eumulgin^{®} B3) | | | | | 14,0 |
| Cetoleth-20 (Eumulgin^{®} 020) | | | | 5,0 | |
| Glycerin, Glyceryl Polyacrylate (Hispagel^{®} 200) | | | 36,7 | | |
| Lauryl Glucoside (Plantacare^{®} 1200 UP) | | | | 5,0 | |
| Laureth-7 Citrate (Plantapon^{®} LC 7) | | 0,7 | 1,0 | | |
| Glycine Soja (Soybean) Sterols (Generol^{®} 122 N) | 0,5 | | | | |
| Hydrogenated Dimer Dilinoleyl / Dimethylcarbonate Copolymer | | | | | |
| (Cosmedia^{®} DC) | 1,0 | | | | |
| Glycerin | 3,0 | | | | |
| Cocamide MEA (Comperlan^{®} 100) | | | | 2,5 | |
| Cetrimonium Chloride (Dehyquart^{®} A) | 3,0 | 4.0 | | | |
| Hydrolyzed Keratin (Nutrilan^{®} Keratin W) | 2,0 | | | | |
| PVPNA (Luvisko^{®}VA 64) | | | 4,5 | | |
| PEG-90M (Polyox^{®} WSR-301) | | | 0,25 | | |
| Hydroxypropyl Methylcellulose (Methocel^{®} E4M Premium EP) | | | 0,6 | | |
| Dicocoylethyl Hydroxyethylmonium Methosulfat, Propylene | | | | | |
| Glycol (Dehyquart^{®} L 80) | | | 0,6 | | |
| Triethanolamine | | | 1,0 | | |
| CaCl₂*2H₂O | | 0,1 | | | |
| Ethanol | | | 12,0 | | |
| Polyquartemium-37 (Ultragel™ 300) | 0,2 | | | | |
| Water, Perfume, Preservatives | q.s. | q.s. | q.s. | q.s. | q.s. |

**Table 17: Rinse-Off Concepts**

| **Ingredients (INCI)** | **1** | **2** | **3** | **4** | **5** | **6** |
|---|---|---|---|---|---|---|
| Sodium Laureth Sulfate (Texapon^{®} N 70) | 12,9 | | 12,3 | 14,3 | 14,3 | |
| Cocamidopropyl Betaine (Dehyton^{®} PK 45) | 7,7 | | 5,4 | 5,4 | 5,4 | |
| Laureth-7 Citrate (Plantapon^{®} LC 7) | 10,0 | 2,5 | | | | 10,0 |
| Guar Hydroxypropyltrimonium Chloride (Cosmedia^{®} Guar C 261 N) | | | 0,25 | 0,2 | | |
| Polyquatemium-7 | | | | 2,5 | | |
| Polyquatemium-10 | | | | | 0,15 | |
| Polyquatemium-44 | | | 1,5 | | 1,5 | |
| Glycol Distearate, Laureth-4, Cocamidopropyl Betaine (Euperlan^{®} PK 4000) , | | | 10.0 | 2,0 | 2,0 | |
| PEG-40 Hydrogenated Castor Oil (Eumulgin^{®} HRE 40) | | 7,5 | | | | |
| Mineral Oil | | | | | | 55,0 |
| (Propylheptyl Caprylate) Cetiol^{®} Sensoft | | | | | | 29,0 |
| **Ester mixture of example 1** | 1.0 | 2,0 | 1,0 | 0,5 | 0,5 | 5,0 |
| Lauryl Alcohol | | | 0.5 | 0.5 | 0,5 | |
| Sodium Chloride | | | adjust viscosity | | | |
| Ethanol | | 25,0 | | | | |
| Water, Perfume, Preservatives | q.s. | q.s. | q.s. | q.s. | q.s. | q.s. |
| pH (adjusted with NaOH or citric acid) | 5,5 | 6,0 | 5,5 | 5,7 | 5,4 | 5,5 |

**Table 18: Rinse-Off Concepts**

| **Ingredients (INCI)** | **7** | **8** | **9** | **10** | **11** | **12** |
|---|---|---|---|---|---|---|
| MIPA-Laureth Sulfate, Laureth-4, Propylene Glycol (Texapon^{®}W 90) | 40,7 | 28,3 | 28,3 | 28,3 | 28,3 | |
| Sodium Laureth Sulfate (Texapon^{®}N 70) | | | | | | 10,9 |
| Coco-Glucoside (Plantacare^{®} 818 UP) | | | | | | 6,9 |
| Laureth-7 Citrate (Plantapon^{®}) LC 7) | 5,0 | 28,3 | 28,3 | 28,3 | 28,3 | |
| Laureth-2 (Mergital^{®} LM2 DEO) | 10,0 | | | | | |
| PEG-7 Glyceryl Cocoate (Getiol^{®} HE) | 1,1 | | | | | |
| Soja Oil | | | 20,7 | | | |
| Almond Oil | | | | | | 0,5 |
| Paraffinum Liquidum | | | | | 7,0 | 23,0 |
| Cyclomethicone ((Dow Coming^{®} 245) | | | | | | |
| Dimethicone Copolyol (Dow Coming^{®} 193) | | | | 1,0 | | |
| Olus (Cegesoft^{®} PS6) | 22,0 | | | | | 10,0 |
| **Ester mixture of example 1** | 20,0 | 41,4 | 20,7 | 40,4 | 34,4 | 15,0 |
| Acrylates Copolymer (Carbopol^{®} Aqua) | | | | | | 4,0 |
| Acrylates/C10-30 Alkyl Acrylate Crosspolymer (Pemulen^{®} TR-1) | | | | | | 0,5 |
| AMP^{®} 95 | 1,2 | | | | | |
| Poloxame^{®} 101 | | 2,0 | 2,0 | 2,0 | 2,0 | |
| Water | | | | | | q.s. |

By exchanging the ester mixture of example 1 by the ester mixtures of examples 2, 3 or 4, the respective corresponding compositions are obtained.

### Appendix - ingredients

Abil^{®} EM 90; INCI: Cetyl Dimethicone Copolyol; Tego Cosmetics (Goldschmidt); Allianz^{®} OPT; INCI: Acrylates/C12-22 Alkyl Methacrylate Copolymer; Rohm und Haas; Amphisol^{®} K; INCI: Potassium Cetyl Phosphate; Hoffmann La Roche; Antaron^{®} V 220; INCI: PVP/Eicosene Copolymer, GAF General Aniline Firm Corp. (IPS-Global); Antaron^{®} V 216; INCI: PVP/Hexadecene Copolymer: GAF General Aniline Firm Corp. (IPS-Global); Arlacel^{®} 83; INCI: Sorbitan Sesquioleate, Uniqema (ICI Surfacants); Arlacel^{®} P 135, INCI: PEG-30 Dipolyhydroxystearate, Uniqema (ICI Surfacants); Bentone^{®} 38, INGI: Quatemium-18 Hectorite, Rheox (Elementis Specialties); Carbopol^{®} 980, INCI: Carbomer, Goodrich; Carbopol^{®} 2984, INCI: Carbomer, Noveon, Inc.; Carbopol^{®} ETD 2001,INCI: Carbomer, Noveon, Inc.; Carbopol^{®} Ultrez 10, INCI: Carbomer; Noveon, Inc.; Cegesoft^{®} C 17, Myristyl Lactate, Cognis GmbH; Cegesoft^{®} PFO, INCI: Passiflora Incamata (EU); Cognis GmbH; Cegesoft^{®} PS 6, INCI: Olus, Cognis GmbH, Ceraphyl^{®} 45, INCI: Diethylhexyl Malate, International Specialty Products ; Cetiol^{®} 868, INCI: Ethylhexyl Stearate, Hersteller: Cognis GmbH; Cetiol^{®} A, INCI: Hexyl Laurate, Cognis GmbH; Cetiol^{®} B, INCI: Dibutyl Adipate, Cognis GmbH; Cetiol^{®} CC, INCI: Dicaprylyl Carbonate; Cognis GmbH; Cetiol^{®} J 600, INCI: Oleyl Erucate, Cognis GmbH; Cetiol^{®} LC, INCI: Coco-Caprylate/Caprate, Cognis GmbH; Cetiol^{®} OE, INGI: Dicaprylyl Ether, Cognis GmbH, Cetiol^{®} PGL, INCI: Hexyldecanol, Hexyldecyl Laurate, Cognis GmbH; Cetiol^{®} S, INCI: Dlethylhexylcyclohexane, Cognis GmbH; Cetiol^{®} SB 45, INCI: Shea Butter Butyrospermum Parkii (Linne), Cognis GmbH; Cetiol^{®} SN, INCI: Cetearyl Isononanoate, Cognis GmbH, Copherol^{®} F 1300 C, INCI: Tocopherol, Cognis GmbH; Copherol 1250 C, INCI: Tocopheryl Acetate, Cognis GmbH; Cosmedia^{®} DC, INCI: Hydrogenated Dimer Dilinoleyl / Dimethylcarbonate Copolymer, Cognis GmbH; Cosmedia^{®} SP, INGI: Sodium Polyacrylate; Cognis GmbH; Cutina^{®} E 24, INCI: PEG-20 Glyceryl Stearate; Cognis GmbH; Cutina^{®} HR, INCI: Hydrogenated Castor Oil, Cognis GmbH; Cutina^{®} MD, INCI:Glyceryl Stearate, Cognis GmbH; Cutina^{®} PES, INCI: Pentaerythrityl Distearate, Cognis GmbH; Dehymuls^{®} FCE, INCI: Dicocoyl Pentaerythrityl Distearyl Citrate, Cognis GmbH; Dehymuls^{®} HRE 7, INCI:PEG-7 Hydrogenated Castor Oil, Cognis GmbH; Dehymuls^{®} PGPH, INCI:Polyglyceryl-2 Dipolyhydroxystearate, Cognis GmbH; Dow Coming^{®} 244 Fluid, INCI: Cyclomethicone, Dow Coming; Dow Corning^{®} 246 Fluid, Cyclopentasiloxane, Dow Coming; Dow Coming^{®} 2502, INCI:Cetyl Dimethicone, Dow Coming; Dry^{®}Flo Plus, INCI:Aluminium Starch Octenylsuccinate, National Starch; Elfacos^{®}ST 37, INCI: PEG-22 Dodecyl Glycol Copolymer, Akzo-Nobel; Effacos^{®}ST 9, INCI:PEG-45 Dodecyl Glycol Copolymer, Akzo-Nobel; Emery^{®} 1780, INCI:Lanolin Alcohol, Cognis Corp.; Emulgade^{®} CM, INCI: Cetearyl Isononanoate and Ceteareth-20 and Cetearyl Alcohol and Glyceryl Stearate and Glycerin and Ceteareth-12 and Cetyl Palmitate, Cognis GmbH; Emulgade^{®}PL 68/50, INCI: Cetearyl Glucoside, Cetearyl Alcohol, Cognis GmbH; Emulgade^{®} SE - PF, INCI: Glyceryl Stearate (and) Ceteareth-20 (and) Ceteareth-12 (and) Cetearyl Alcohol (and) Cetyl Palmitate; Cognis GmbH, Emulgade^{®}SUCRO, INCI: Sucrose Polystearate (and) Hydrogenated Polyisobutene, Cognis GmbH; Eumulgin^{®}B1, INGI: Ceteareth-12, Cognis GmbH, Eumulgin^{®} B 2, INCI: Ceteareth- 20, Cognis GmbH; Eumulgin^{®}HRE 40, INCI: PEG-40 Hydrogenated Castor Oil, Cognis GmbH; Eumulgin^{®}SG, INCI: Sodium Stearoyl Glutamate, Cognis GmbH; Eumulgin^{®} VL 75, INCI: Lauryl Glucoside (and) Polyglyceryl-2 Dipolyhydroxystearate (and) Glycerin; Cognis GmbH; Eusolex^{®} OCR, INCI: Octocrylene, Merck; Eusolex^{®} T 2000, INCI: Titanium Dioxide, Alumina, Simethicone, Merck; Eusotex^{®}T AQUA, INCI: Water and Titanium Dioxide and Alumina and Sodium Metaphosphate and Phenoxyethanol and Sodium Methylparaben, Merck; Eutanol^{®}G, INCI: Octyldodecanol, Cognis GmbH; Eutanol^{®}G 16, INCI: Hexyldecanol, Cognis GmbH; Eutanol^{®}G 16 S, INCI: Hexyldecyl Stearate, Cognis GmbH; Finsolv^{®} TN, INCI: C 12/15 Alkyl Benzoate, Findex (Nordmann/Rassmann); Generol^{®} R, INCI: Brassica Campestris (Rapseed) Sterols, Cognis GmbH; Glucate^{®}DO, INCI: Methyl Glucose Dioleate, NRC Nordmann/Rassmann; Hispagel^{®} 200, INCI: Glycerin, Glyceryl Polyacrylate, Cognis GmbH; Hostaphat^{®} KL 340 N, INCI: Trilaureth-4 Phosphate, Clariant; Hydagen^{®} C.A.T.,INCI Triethyl Citrate, Cognis GmbH; Hydagen^{®}DCMF, INCI: Chitosan, Cognis GmbH; Insect Repellent^{®}3535, INCI: Ethyl Butylacetylaminopropionate, EMD Chemicals Inc; Isolan^{®}PDI, INCI: Diisostearoyl Polyglyceryl-3 Diisostearate, Goldschmidt AG; Keltrol^{®}T, INCI: Xanthan Gum, CP Kelco; Lameform^{®}TGI, INCI: Polyglyceryl-3 Diisostearate, Cognis GmbH; Lanette^{®}14. INCI: Myristyl Alcohol, Cognis GmbH; Lanette^{®}18, INCI: Stearyl Alcohol, Cognis GmbH; Lanette^{®}22, INCI: Behenyl Alcohol, Cognis GmbH; Lanette^{®}E, INCI: Sodium Cetearyl Sulfate, Cognis GmbH; Lanette^{®}O, INCI: Cetearyl Alcohol, Cognis GmbH; Locron^{®} L, INCI: Aluminium Chlorhydrate, Clariant; Lucentite^{®} SAN, INCI: Quatemium-18 Hectorit, Co-Op Chemical Co., Ltd.; Monomuls^{®} 90-018, INCI: Glyceryl Oleate, Cognis GmbH; Myrj^{®} 51, INCI: PEG-30-Sterate, Uniqema; Mynitol^{®} 312, INCI: Caprylic/Capric Triglyceride, Cognis GmbH; Myritol^{®}331, INGI: Cocoglycerides, Cognis GmbH; Myritol^{®}PC, INCI:PropyleneGlycol Dicaprylate/ Dicaprate, Cognis GmbH; Neo Heliopan^{®} 303, INGI: Octocrylene, Symrise; Neo Heliopan^{®}AP, INCI: Disodium Phenyl Dibenzimidazole Tetrasulfonate, Symrise; Neo Heliopa^{®}AV, INCI: Ethylhexyl Methoxycinnamate, Symrise; Neo Heliopan^{®} BB, INCI: Benzophenone-3, Symrise; Neo Heliopan^{®} E 1000, INCI: Isoamyl-p-Methoxycinnamate, Symrise; Neo Heliopan^{®}Hydro, INCI: Phenylbenzimidazole Sulfonic Acid, Symrise; Neo Heliopan^{®} MBC, INCI: 4-Methylbenzylidene Camphor, Symrise; Neo Heliopan^{®} OS, INCI: Ethylhexyl Salicylate, Symrise; Novata^{®} AB, INCI: Cocoglycerides, Cognis GmbH; Parsol^{®} 1789, INCI: Butyl Methoxydibenzoylmethane, Hoffmann-La Roche (Givaudan); Pemulen^{®} TR-2 Polymer, INGI: Acrylates / C10-30 Alkylacrylate Crosspolymer, Noveon, Inc.; Photonyl^{®}LS, INCI: Arginine, Disodium Adenosine Triphosphate, Mannitol, Pyridoxine HCL, Phenylalanine, Tyrosine, Laboratoires Serobiologiques (Cognis); Prisorine^{®} 3505, INCI: Isostearic Acid; Uniqema; Prisorine^{®} 3758, INCI: Hydrogenated Polyisobutene, Uniqema; Rezal 36G, INCI: Aluminum Zirconium Tetrachlorohydrex GLY, Reheis, Inc; Rheocare^{®} C Plus, INCI Carbomer, Cognis GmbH; SFE^{®}839, INCI: Cyclopentasiloxane and Dimethicone/Vinyl Dimethicone Crosspolymer, GE Silicones; Silikonöl Wacker AK^{®}350, INCI: Dimethicone, Wacker, Tego^{®}Care 450, INCI: Polyglyceryl-3 Methylglucose Distearate, Goldschmidt; Tego^{®}Care CG 90, INCI: Cetearyl Glucoside, Goldschmidt; Tegosoft^{®} DEC, INCI: Diethylhexyl Carbonate, Goldschmidt; Tinosorb^{®} S, INGI: Bis-Ethylhexyloxyphenol Methoxyphenyl Triazine; Ciba Specialty Chemicals Corporation; Tinosorb^{®} M, INGI: Methylene Bis-Benzotriazolyl Tetramethylbutylphenol, Ciba Specialty Chemicals Corporation; Tween^{®} 60, INCI: Polysorbate 60, Uniqema (ICI Surfactants), Uvasorb^{®} HEB, INCI: Diethylhexyl Butamido Triazone, 3V Inc.; Unirep^{®} U-18, INCI: Dimethyl Phthalate and Diethyl Toluamide and Ethyl Hexanediol, Induchem AG; Uvinul^{®} T 150, INCI: Ethylhexyl Triazone, BASF; Uvinul^{®} A plus, INCI: Diethylamino Hydroxybenzoyl Hexyl Benzoate, BASF; Veegum^{®} Ultra, INCI: Magnesium Aluminium Silicate, R. T. Vanderbilt Company, Inc; Veegum^{®} Plus, INCI: Magnesium Aluminum Silicate and Cellulose Gum, R. T. Vanderbilt Company, Inc; Z-Cote^{®} HP 1, INCI: Zinc Oxide and Triethoxy-caprylylsilane, BASF, Zinc Oxide NDM, INCI: Zinc Oxide, Symrise.

## Claims

1. Mixture of esters according to the general formula (I),
R₁-C(=O)-O-R₂
wherein R₁ is an alkyl moiety with 7 to 9 carbon atoms and wherein R₂ is a an alkyl moiety with 8 to 10 carbon atoms ,
wherein the mixture comprises 5 to 60 weight-% of ester of the general formula (I), wherein **R₁** is an **alkyl moiety with 9 carbon atoms,** based on the total amount of esters according to formula (I)
and/or wherein the mixture comprises 5 to 60 weight-% of ester of the general formula (I), wherein **R₂** is an alkyl moiety with 10 carbon atoms, based on the total amount of esters according to formula (I).

2. Mixture of esters according to claim 1, wherein R₁ comprises 5 to 20 weight-% of ester of the general formula (I), wherein R₁ is an alkyl **moiety with 9 carbon atoms,** based on the total amount of esters according to formula (I).

3. Mixture of esters according to any preceding claim, wherein the mixture comprises 5 to 20 weight-% of ester of the general formula (I), wherein **R₂** is an **alkyl moiety with 10 carbon atoms,** based on the total amount of esters according to formula (I).

4. Mixture of esters according any preceding claim, wherein the R₁ and/or R₂ is a linear and/or unsaturated alkyl moiety.

5. Mixture of esters according to any preceding claim, wherein the amount of branched esters is 50 or less than 50 weight-%, preferably 25 or less than 25 weight-%, preferably 10 or less than 5 weight-%, more preferably 5 or less than 5 weight-%, most preferably 1 or less than 1 weight.-%, based on the total amount of esters according to formula (I).

6. Process for the production of ester mixtures of formula (I), R₁-C(=O)-O-R₂ wherein a carbon acid or a mixture of carbons acids R₁-COOH is reacted with an alcohol or a mixture of alcohols R₂-OH, wherein R₁ is an alkyl moiety with 7 to 9 carbon atoms and wherein R₂ is a an alkyl moiety with 8 to 10 carbon atoms, in such a ratio, that the resulting ester mixture comprises 5 to 60 weight-% of ester of the general formula (I), wherein **R₁** is an **alkyl moiety with 9 carbon atoms,** based on the total amount of esters according to formula (I) and/or the mixture comprises 5 to 60 weight-% of ester of the general formula (I), wherein **R₂** is an **alkyl moiety with 10 carbon atoms**, based on the total amount of esters according to formula (I).

7. Process for the production of ester mixtures of formula (I), R₁-C(=O)-O-R₂ wherein a carbon acid methyl ester or a mixture of carbon acid methyl esters R₁-C(=O)-CH₃ are reacted with an alcohol or a mixture of alcohols R₂-OH, wherein R₁ is an alkyl moiety with 7 to 9 carbon atoms and wherein R₂ is a an alkyl moiety with 8 to 10 carbon atoms in the presence of a transesterification catalyst, in such a ratio, that the resulting ester mixture comprises 5 to 60 weight-% of ester of the general formula (I), wherein R₁ is an **alkyl moiety with 9 carbon atoms,** based on the total amount of esters according to formula (I) and/or the mixture comprises 5 to 60 weight-% of ester of the general formula (I), wherein **R₂ is an alkyl moiety with 10 carbon atoms,** based on the total amount of esters according to formula (1).

8. Cosmetic and/or pharmaceutical composition comprising 0,1 to 95 weight-% of a mixture of esters according to the general formula (I),
R₁-C(=O)-O-R₂
wherein R₁ is an alkyl moiety with 7 to 9 carbon atoms and wherein R₂ is a an alkyl moiety with 8 to 10 carbon atoms,
wherein the mixture comprises 5 to 60 weight-% of ester of the general formula (I), wherein **R₁ is an alkyl moiety with 9 carbon atoms,** based on the total amount of esters according to formula (I).
and/or wherein the mixture comprises 5 to 60 weight-% of ester of the general formula (I), wherein **R₂ is an alkyl moiety with 10 carbon atoms,** based on the total amount of esters according to formula (I).

9. Use of an ester mixture according to any one of claims 1 to 5 for the preparation of or in cosmetic and/or pharmaceutical compositions.

10. Use according to claim 9 as oil component and/or solubiliser.
